# EUROPEAN PATENT APPLICATION

(11) **EP 2 236 507 A1**
(43) Date of publication of application: **06.10.2010**
(21) Application number: 08864124.6
(22) Date of filing: 15.12.2008
(51) Int. Cl.: C07D 401/14, A61K 31/4439, A61K 31/444, A61K 38/22, A61K 38/28, A61K 45/00, A61P 3/04, A61P 3/10, A61P 43/00

(54) **N-PYRAZOLE-2-PYRIDINECARBOXAMIDE DERIVATIVE**

(30) Priority: 25.12.2007 JP 2007331340
(71) Applicant: Banyu Pharmaceutical Co., Ltd., Chiyoda-ku Tokyo 102-8667 (JP)
(72) Inventor: SAGARA, Yufu, Tsukuba-shi Ibaraki 300-2611 (JP); HASHIMOTO, Noriaki, Tsukuba-shi Ibaraki 300-2611 (JP); NISHIMURA, Teruyuki, Tsukuba-shi Ibaraki 300-2611 (JP)
(74) Representative: Buchan, Gavin MacNicol
(86) International application number: PCT/JP2008/072750
(87) International publication number: WO 2009/081782

(57) **Abstract**

The present invention relates to a compound represented by the formula: 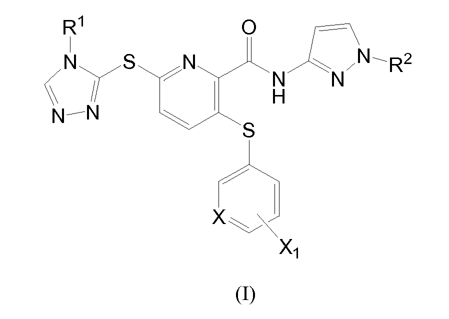 or a pharmaceutically acceptable salt thereof, wherein R¹ and R² are each independently a lower alkyl group, X is CH or the like, and X₁ is an aminoalkoxy group or the like, which has a glucokinase-activating effect and thus is useful in the treatment of diabetes, obesity, and the like.

## Description

### TECHNICAL FIELD

The present invention relates to a glucokinase activator containing an N-pyrazole-2-pyridinecarboxamide derivative as an active ingredient. The present invention further relates to a novel N-pyrazole-2-pyridinecarboxamide derivative.

### BACKGROUND ART

Glucokinase (GK) (ATP: D-hexose 6-phosphotransferase, EC 2.7.1.1) is one of four mammalian hexokinases (hexokinase IV). Hexokinases are enzymes in the first step of the glycolytic pathway and catalyze the reaction from glucose to glucose-6-phosphate. Glucokinase is expressed principally in the liver and pancreatic beta cells and plays an important role in whole-body glucose metabolism by controlling the rate-determining step in glucose metabolism in these cells. The glucokinases expressed in the liver and pancreatic beta cells differ in the sequence of the 15 N-terminal amino acids due to a difference in splicing, respectively, whereas their enzymatic characteristics are identical. The enzyme activities of the three hexokinases (I, II, and III) other than the glucokinase become saturated at a glucose concentration of 1 mM or lower, whereas the Km of glucokinase to glucose is 8 mM, which is close to the physiological blood glucose level. Accordingly, glucokinase-mediated intracellular glucose metabolism is accelerated in response to blood glucose level changes by postprandial glucose level increase (10-15 mM) from normal glucose (5 mM).
It has been hypothesized for around 10 years that glucokinase serves as a glucose sensor for pancreatic beta cells and the liver (for example, see non patent document 1. Recent results in glucokinase gene-manipulated mice have confirmed that glucokinase does in fact play an important role in systemic glucose homeostasis. Mice lacking a functional glucokinase gene die shortly after birth (for example, see non patent document 2, while healthy and diabetic mice overexpressing glucokinase have lower blood glucose levels (for example, see non patent document 3). With glucose level increase, the reactions of pancreatic beta- and liver cells, while differing, both act toward lowering blood glucose. Pancreatic beta cells secrete more insulin, while the liver takes up glucose and stores it as glycogen while also reducing glucose release.
Such variation in glucokinase enzyme activity is important for liver and pancreatic beta cell-mediated glucose homeostasis in mammals. A glucokinase gene mutation has been found in a case of diabetes which occurs in youth, referred to as MODY2 (maturity-onset diabetes of the young), and the reduced glucokinase activity has been shown to be responsible for blood glucose increase (for example, non patent document 4). In contrast, families having a mutation increasing the glucokinase activity has been found, and such individuals exhibit hypoglycemia (for example, see non patent document 5).
These suggest that in humans as well, glucokinase functions as a glucose sensor and thus plays an important role in glucose homeostasis. Glucose regulation utilizing a glucokinase sensor system is likely to be possible to achieve in most patients with type II diabetes mellitus. Since glucokinase activators should have effects of accelerating insulin secretion by pancreatic beta cells and of promoting glucose uptake and inhibiting glucose release by the liver, they are likely to be useful as therapeutic agents for patients with type II diabetes mellitus.
In recent years, it has been found that pancreatic beta cell glucokinase is expressed locally in rat brain, particularly in the ventromedial hypothalamus (VMH). Around 20% of VMH neurons are referred to as "glucose-responsive neurons", and these have long been considered to play an important role in body weight control. Administration of glucose into rat brain reduces feeding consumption, whereas inhibition of glucose metabolism by intracerebral administration of glucose analog glucosamine produces hyperphagia. Electrophysiological experiments have indicated that glucose-responsive neurons are activated in response to physiological glucose level changes (5-20 mM) but that their activation is inhibited with glucose metabolism inhibition by, e.g., glucosamine. The glucose level-detecting system in the VMH is intended to be based on a glucokinase-mediated mechanism similar to that for insulin secretion by pancreatic beta cells. Accordingly, substances which activate glucokinase in the VMH in addition to the liver and pancreatic beta cells not only exhibit a glucose rectifying effect but can also potentially rectify obesity, which is a problem for most patients with type II diabetes mellitus.
The above description indicates that compounds having glucokinase-activating effects are useful as therapeutic and/or prophylactic agents for diabetes mellitus, as therapeutic and/or prophylactic agents for chronic complications of diabetes mellitus, such as retinopathy, nephropathy, neurosis, ischemic heart disease and arteriosclerosis, and further as therapeutic and/or prophylactic agents for obesity.
As a compound related to the N-pyrazole-2-pyridinecarboxamide derivative according to the present invention, for example, Patent Document 1 discloses a compound represented by the below formula:

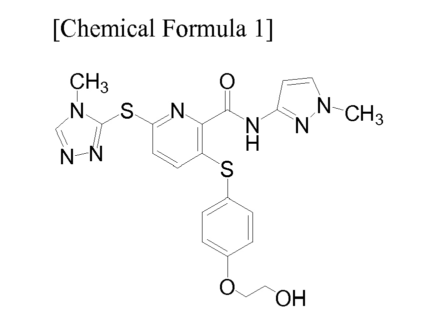

Patent Document 1: WO 2004/081001
Non-patent Document 1: Garfinkel D. et al., Computer modeling identifies glucokinase as glucose sensor of pancreatic beta-cells, American Journal Physiology, Vol. 247 (3Pt2), 1984, pp. 527-536
Non-patent Document 2: Grupe A. et al., Transgenic knockouts reveal a critical requirement for pancreatic beta cell glucokinase in maintaining glucose homeostasis, Cell, Vol. 83, 1995, pp. 69-78
Non-patent Document 3: Ferre T. et al., Correction of diabetic alterations by glucokinase, Proceedings of the National Academy of Sciences of the U.S.A., Vol. 93, 1996, pp. 7225-7230
Non-patent Document 4: Vionnet N. et al., Nonsense mutation in the glucokinase gene causes early-onset non-insulin-dependent diabetes mellitus, Nature Genetics, Vol. 356, 1992, pp. 721-722
Non-patent Document 5: Glaser B. et al., Familial hyperinsulinism caused by an activating glucokinase mutation, New England Journal Medicine, Vol. 338, 1998, pp. 226-230

### DISCLOSURE OF THE INVENTION

### PROBLEM TO BE SOLVED BY THE INVENTION

An object of the present invention is to provide a therapeutic agent and/or prophylactic agent for diabetes, which binds to glucokinase to increase the activity of glucokinase, and also provide an anti-obesity agent that acts by activating glucokinase to thereby stimulate the satiety center. Another object is to provide a compound with medicinal properties and/or improved physical properties as a medicine.

### MEANS FOR SOLVING THE PROBLEMS

The present inventors conducted intensive research, as a result, they found that a compound represented by the following Formula (I) or a pharmaceutically acceptable salt thereof:

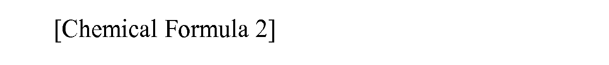

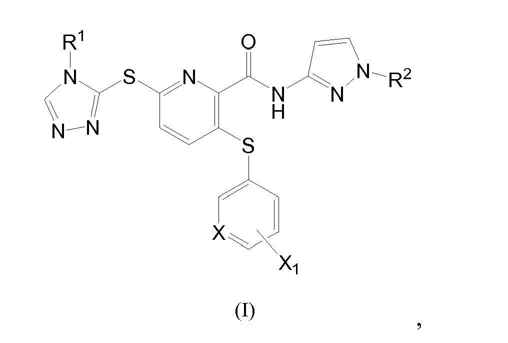

wherein:
R¹ and R² are each independently a lower alkyl group, X is CH or a nitrogen atom, and
X₁ is a group represented by Formula (II-1):

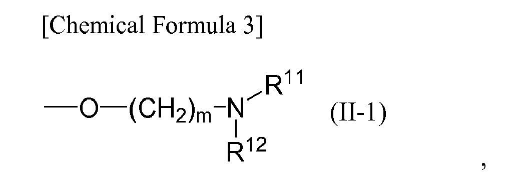

wherein R¹¹ and R¹² are each independently a hydrogen atom or a lower alkyl group; R¹¹, R¹², and the nitrogen atom to which they are bound together form a 4- to 7-membered nitrogen-containing aliphatic ring, wherein one of the carbon atoms that form the 4- to 7-membered nitrogen-containing aliphatic ring may be substituted with an oxygen atom; or alternatively, an arbitrary carbon atom in (CH₂)ₘ and R¹¹ or R¹²
together form a 4- to 7-membered nitrogen-containing aliphatic ring, wherein the 4- to 7-membered nitrogen-containing aliphatic ring may be substituted with an oxo group, the nitrogen atom to which R¹¹ and R¹² are bound each other has an oxygen atom added thereto, an arbitrary carbon atom in (CH₂)ₘ may be substituted with a lower alkyl group, and m is an integer of 1 to 3; or
a group represented by Formula (II-2):

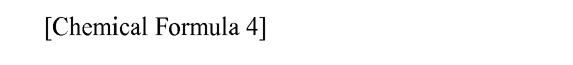

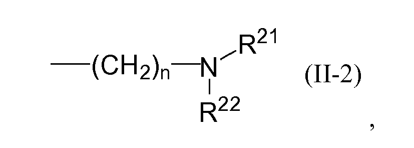

wherein R²¹ and R²² are each independently a hydrogen atom or a lower alkyl group; or R²¹, R²², and the nitrogen atom to which they are bound to together form a 4- to 7-membered nitrogen-containing aliphatic ring, wherein the 4- to 7-membered nitrogen-containing aliphatic ring may be substituted with an oxo group, an arbitrary carbon atom in (CH₂)ₙ may be substituted with a lower alkyl group, and n is an integer of 0 or 1, has greatly improved solubility such as physical properties and/or medicinal properties over a conventional 2-pyridinecarboxamide derivative, and thus accomplished the present invention.

### EFFECTS OF THE INVENTION

The N-pyrazole-2-pyridinecarboxamide derivative represented by Formula (I) according to the present invention or a pharmaceutically acceptable salt thereof has a strong glucokinase-activating effect, and is useful in the treatment and/or prevention of diabetes, diabetic complication, or obesity. Further, as a medicine, the N-pyrazole-2-pyridinecarboxamide derivative according to the present invention is superior to a conventional 2-pyridinecarboxamide derivative in terms of solubility such as physical properties and/or medicinal properties.
The compound of the present invention is applicable to both types of diabetes, insulin dependent diabetes mellitus (IDDM) and non-insulin dependent diabetes mellitus (NIDDM).
A diabetic complication herein is a disease that occurs with development of diabetes, and specific examples of diabetic complications include diabetic nephropathy, diabetic retinopathy, diabetic neurosis, diabetic arteriosclerosis, and the like.

### BEST MODE FOR CARRYING OUT THE INVENTION

Hereinafter, the terms used herein will be explained, and the compound according to the present invention will be explained in further detail.
A "halogen atom" is, for example, a fluorine atom, a chlorine atom, a bromine atom, an iodine atom, or the like.
A "lower alkyl group" is a C₁₋₆ straight or branched alkyl group, including such as a methyl group, an ethyl group, a propyl group, an isopropyl group, a butyl group, an isobutyl group, a sec-butyl group, a tert-butyl group (also called t-butyl), a pentyl group, an isoamyl group, a neopentyl group, an isopentyl group, a 1,1-dimethylpropyl group, a 1-methylbutyl group, a 2-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,2,2-trimethylpropyl group, a 1-ethyl-2-methylpropyl group.
An "alkoxy group" is a group wherein the hydrogen atom of the hydroxyl group is substituted with the lower alkyl group, including such as a methoxy group, an ethoxy group, a propoxy group, an isopropoxy group, a butoxy group, a sec-butoxy group, a tert-butoxy group, a pentyloxy group, an isopentyloxy group, a hexyloxy group, an isohexyloxy group.
For more specific disclosure of a compound represented by Formula (I) of the present invention:

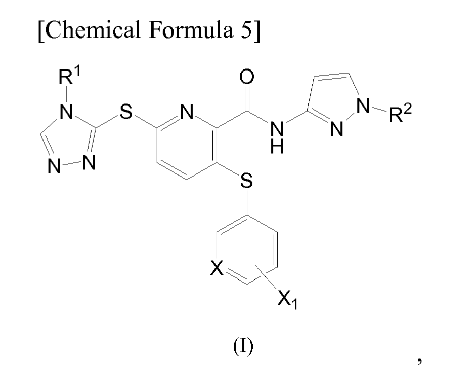

wherein the symbols are as defined above, the symbols used in Formula (I) are explained through specific examples.
R¹ and R² are each independently a lower alkyl group.
As the "lower alkyl group" represented by R¹ and R², a group same as the "lower alkyl group" defined above can be mentioned, and among these, R¹ and R² are preferably each independently a methyl group, an ethyl group, n-propyl group, or an isopropyl group. More preferably, R¹ and R² are each independently a methyl group or an ethyl group, and especially preferably, R¹ and R² are both methyl groups.
X₁ is a group represented by Formula (II-1):

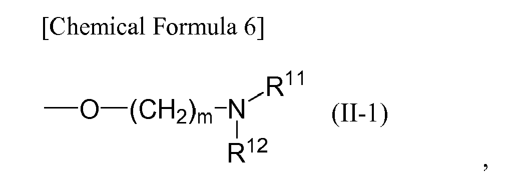

wherein the symbols are as defined above, or Formula (II-2):

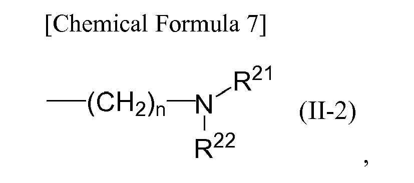

wherein the symbols are as defined above.
Groups represented in Formula (II-1) are explained.
R¹¹ and R¹² are each independently a hydrogen atom or a lower alkyl group.
The "lower alkyl group" represented by R¹¹ and R¹² is a group same as the "lower alkyl group" defined above, specific examples thereof including such as a methyl group, an ethyl group, an isopropyl group, an n-propyl group.
In the case where R¹¹ and R¹² are each independently a hydrogen atom or a lower alkyl group, specific examples of groups represented by the formula:

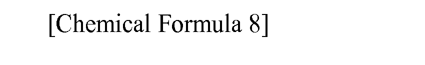

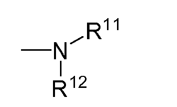

Include such as an amino group, a methylamino group, a dimethylamino group, an ethylamino group, a diethylamino group, an ethyl methylamino group.
In the case where R¹¹ and R¹² are each independently a hydrogen atom or a lower alkyl group, the nitrogen atom to which R¹¹ and R¹² are bound each other may have an oxygen atom added thereto.
Specific examples of groups represented by the formula:

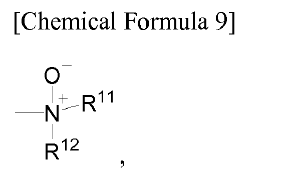

wherein an oxygen atom is added, include a dimethylnitroryl group, a diethylnitroryl group, an ethylmethylnitroryl, and the like.
R¹¹, R¹², and the nitrogen atom to which they are bound may together form a 4- to 7-membered nitrogen-containing aliphatic ring, and one of the carbon atoms that form the 4- to 7-membered nitrogen-containing aliphatic ring may be substituted with an oxygen atom.
When R¹¹, R¹², and the nitrogen atom to which they are bound to together form a 4- to 7-membered nitrogen-containing aliphatic ring, the bond may be formed at any position where R¹¹ and R¹² can be bound.
Further, either R¹¹ or R¹² may alternatively form, together with an arbitrary carbon atom in (CH₂)ₘ of the Formula (II-1), a 4- to 7-membered nitrogen-containing aliphatic ring.
When R¹¹, R¹², and the nitrogen atom to which they are bound together form a 4- to 7-membered nitrogen-containing aliphatic ring, wherein one of the carbon atoms that form the ring may be substituted with an oxygen atom, the "4- to 7-membered nitrogen-containing aliphatic ring" may specifically be, for example, an azetidin-1-yl group, a pyrrolidin-1-yl group, a (2R)-2-methylpyrrolidin-1-yl group, a (2S)-2-methylpyrrolidin-1-yl group, a piperidin-1-yl group, a hexamethyleneimin-1-yl group, a morpholin-4-yl group, and the like.
When either R¹¹ or R¹² forms, together with an arbitrary carbon atom in (CH₂)ₘ, a 4- to 7-membered nitrogen-containing aliphatic ring, the 4- to 7-membered nitrogen-containing aliphatic ring may specifically be, for example, a 1-methylazetidin-3-yl group, a 1-ethylazetidin-3-yl group, a 1-isopropylazetidin-3-yl group, a 1-isopropylpyrrolidin-3-yl group, a 1-methylpyrrolidin-2-yl group, a pyrrolidin-3-yl group, a 1-methylpyrrolidin-3-yl group, a 1-ethylpyrrolidin-3-yl group, a 1-methylpiperidin-4-yl group, and the like.
In Formula (II-1), when R¹¹, R¹², and the nitrogen atom to which they are bound to in the formula:

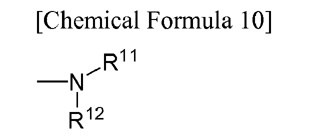

together form a 4- to 7-membered nitrogen-containing aliphatic ring, or when an arbitrary carbon atom in (CH₂)ₘ and R¹¹ or R¹² in the formula:

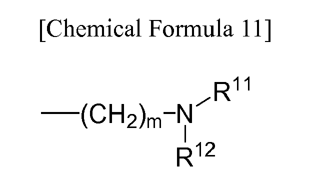

together form a 4- to 7-membered nitrogen-containing aliphatic ring, such a 4- to 7-membered nitrogen-containing aliphatic ring may be substituted with an oxo group, and the nitrogen atom that forms the 4- to 7-membered nitrogen-containing aliphatic ring may have an oxygen atom add thereto.
Examples of 4- to 7-membered nitrogen-containing aliphatic rings substituted with an oxo group include a 2-oxopyrrolidin-1-yl group, a 2-oxopiperidin-1-yl group, a 2-oxohexamethyleneimin-1-yl group, and the like.
Specific examples of 4- to 7-membered nitrogen-containing aliphatic rings having the oxygen atom added thereto include a 2-methyl-1-oxidepyrrolidin-1-yl group and the like.
The arbitrary carbon atom in (CH₂)ₘ may be substituted with a lower alkyl group as defined above.
m is an integer of 1 to 3.

Accordingly, specific examples of groups represented by Formula (II-1) include a (1-methylazetidin-3-yl)oxy group, a (1-ethylazetidin-3-yl)oxy group, a (1-isopropylazetidin-3-yl)oxy group, a 2-azetidin-1-ylethoxy group, a 2-pyrrolidin-1-ylethoxy group, a 2-(2-methylpyrrolidin-1-yl)ethoxy group, a 2-((2S)-methylpyrrolidin-1-yl)ethoxy group, a 2-((2R)-2-methylpyrrolidin-1-yl)ethoxy group, a pyrrolidin-3-yloxy group, a (3R)-pyrrolidin-3-yloxy group, a (1-methylpyrrolidin-2-yl)methoxy group, a ((2R)-1-methylpyrrolidin-2-yl)methoxy group, a ((2S)-1-methylpyrrolidin-2-yl)methoxy group, a (1-methylpyrrolidin-3-yl)methoxy group, a ((3S)-1-methylpyrrolidin-3-yl)methoxy group, a ((3S)-1-methylpyrrolidin-3-yl)methoxy group, a (1-methylpyrrolidin-3-yl)oxy group, a ((3S)-1-methylpyrrolidin-3-yl)oxy group, a ((3R)-1-methylpyrrolidin-3-yl)oxy group, a pyrrolidin-3-yloxy group, a (1-isopropylpyrrolidin-3-yl)oxy group, a 1-ethylpyrrolidin-3-yloxy group, a ((3R)-1-ethylpyrrolidin-3-yl)oxy group, a 2-(2-oxopyrrolidin-1-yl)ethoxy group, a (1-methylpiperidin-4-yl)oxy group, a 2-piperidin-1-ylethoxy group, a 2-(diethylamino)ethoxy group, a 2-(dimethylamino)ethoxy group, a 2-(ethylmethylamino)ethoxy group, a 2-(methylamino)ethoxy group, a 2-aminoethoxy group, a 3-pyrrolidin-1-ylpropoxy group, a 3-(dimethylamino)-propoxy group, a 2-morpholin-4-ylethoxy group, a 2-(dimethylnitroryl)ethoxy group, a 2-(2-methyl-1-oxide pyrrolidin-1-yl)ethoxy group, a 2-((2R)2-methyl-1-oxide pyrrolidin-1-yl)ethoxy group, and the like, and among these, a 2-(dimethylamino)ethoxy group, a 2-(diethylamino)ethoxy group, a 2-pyrrolidin-1-ylethoxy group, a (1-methylazetidin-3-yloxy group, a (1-ethylazetidin-3-yl)oxy group, a 1-isopropylazetidin-3-yl)oxy group, a (1-ethylazetidin-3-yl)methoxy group, and a (1-isopropylazetidin-3-yl)methoxy group are preferable.

Next, groups represented in Formula (II-2) are explained.
R²¹ and R²² are each independently a hydrogen atom or a lower alkyl group, and alternatively, R²¹, R²², and the nitrogen atom to which they are bound to may together form a 4- to 7-membered nitrogen-containing aliphatic ring, wherein the 4- to 7-membered nitrogen-containing aliphatic ring may be substituted with an oxo group.

An arbitrary carbon atom in (CH₂)ₙ in Formula (II-2) may be substituted with a lower alkyl group.
n is an integer of 0 or 1.
A specific example of a group represented by Formula (II-2) is a (2-oxopyrrolidin-1-yl)methyl group or the like.
Among the compounds represented by Formula (I), a compound represented by Formula (I-1) or a pharmaceutically acceptable salt thereof:

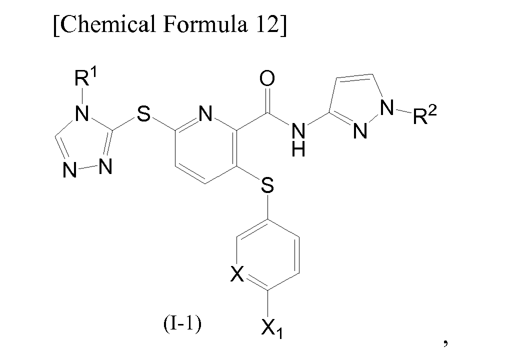

wherein the symbols are as defined above, is preferable, and further, a compound represented by Formula (1-2) or a pharmaceutically acceptable salt thereof:

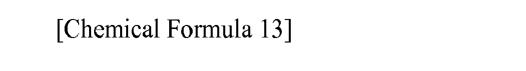

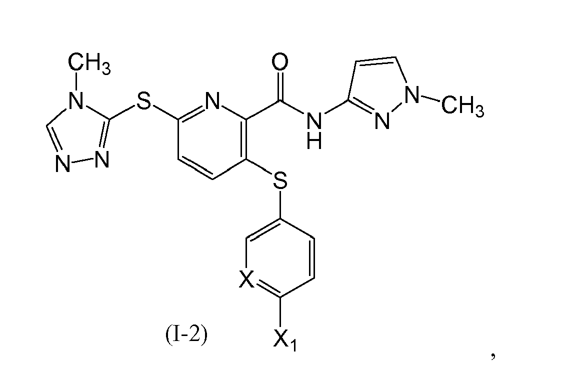

wherein the symbols are as defined above, is more preferable.
As X₁, a group represented by Formula (II-1):

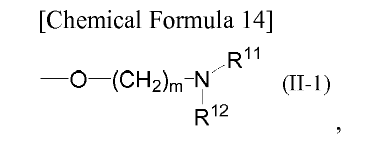

wherein the symbols are as defined above, is preferable.
X is CH or a nitrogen atom.
Among the compounds represented by Formula (I-1), a compound wherein X is CH and X₁ has Formula (II-1-1):

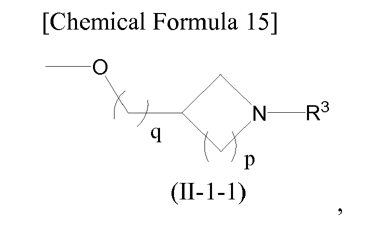

wherein R³ is a lower alkyl group, p is an integer of 1 or 2, and q is an integer of 0 to 2, or a pharmaceutically acceptable salt thereof is preferable.
Among the groups represented by the Formula (II-1-1), one wherein p is 1 and q is 0 or 1 is preferable.
As preferable embodiments of the above-explained R¹, R², R³, R¹¹, R¹², R²¹, R²², X, X₁, m, n, p, and q, any combination may be employed.
Specific examples of compounds represented by Formula (I) include:
3-({4-[2-(dimethylamino)ethoxy]phenyl}thio)-N-(1-methyl-1H-pyrazol-3-yl)-6-[(4-methyl-4H-1,2,4-triazol-3-yl)thio]pyridine-2-carboxamide,
3-({4-[2-(diethylamino)ethoxy]phenyl}thio)-N-(1-methyl-1H-pyrazol-3-yl)-6-[(4-methyl-4H-1,2,4-triazol-3-yl)thio]pyridine-2-carboxamide,
N-(1-methyl-1H-pyrazol-3-yl)-6-[(4-methyl-4H-1,2,4-triazol-3-yl)thio]-3-{[4-(2-pyrrolidin-1-ylethoxy)phenyl]thio}pyridine-2-carboxamide,
3-({4-[(1-methylazetidin-3-yl)oxy]phenyl}thio)-N-(1-methyl-1H-pyrazol-3-yl )-6-[(4-methyl-4H-1,2,4-triazol-3-yl)thio]pyridine-2-carboxamide,
3-((4-[(1-ethylazetidin-3-yl)oxy]phenyl)thio)-N-(1-methyl-1H-pyrazol-3-yl)-6-[(4-methyl-4H-1,2,4-triazol-3-yl)thio]pyridine-2-carboxamide,
3-({4-[(1-isopropylazetidin-3-yl)oxy]phenyl}thio)-N-(1-methyl-1H-pyrazol-3 -yl)-6-[(4-methyl-4H-1,2,4-triazol-3-yl)thio]pyridine-2-carboxamide,
3-({4-[(1-ethylazetidin-3-yl)methoxy]phenyl}thio)-N-(1-methyl-1H-pyrazol-3 -yl)-6-[(4-methyl-4H-1,2,4-triazol-3-yl)thio]pyridine-2-carboxamide,
3-({4-[(1-isopropylazetidin-3-yl)methoxy]phenyl}thio)-N-(1-methyl-1H-pyra zol-3-yl)-6-[(4-methyl-4H-1,2,4-triazol-3-yl)thio]pyridine-2-carboxamide, or
3-({6-[2-(dimethylamino)ethoxy]pyridin-3-yl}thio)-N-(1-methyl-1H-pyrazol-3-yl)-6-[(4-methyl-4H-1,2,4-triazol-3-yl)thio]pyridine-2-carboxamide, and the like.

Hereinafter, a method for producing the compound according to the present invention is explained.

A compound represented by Formula (I) according to the present invention:

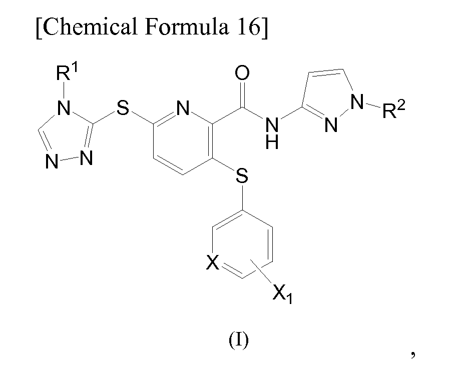

wherein the symbols are as defined above, can be produced by the following method, for example.

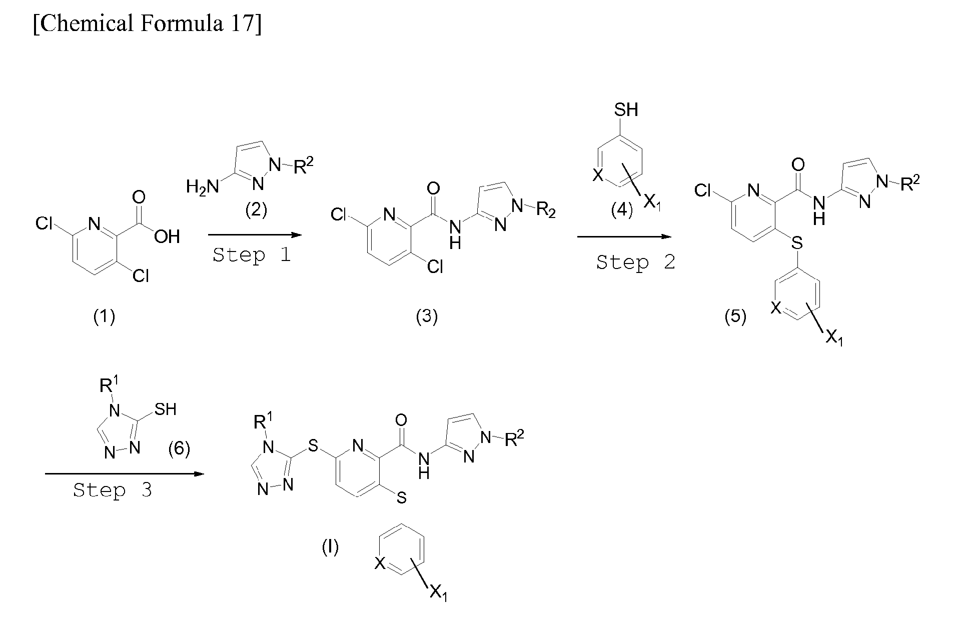

In the formulae, the symbols are as defined above.

### (Step 1)

This step is a method of reacting dichloropyridine carboxylic acid (1) or a reactive derivative thereof with an amino compound (2) to produce a compound (3).

This reaction may be an ordinary amide-forming reaction by a method described in references (e.g., Peptide Gosei no Kiso to Jikken (Basics and Experiments of Peptide Synthesis), Nobuo Izumiya et al., Maruzen, 1983; Comprehensive Organic Synthesis, Vol. 6, Pergamon Press, 1991; etc), a method based on the same, or a combination of such methods with an ordinary method, that is, the reaction may be performed using a condensing agent well known to those skilled in the art, or alternatively by an ester activation method, a mixed acid anhydride method, an acid chloride method, a carbodiimide method, or the like available to those skilled in the art. Examples of such amide-forming reagents include thionyl chloride, oxalyl chloride, N,N-dicyclohexyl carbodiimide, 1-methyl-2-bromopyridinium iodide, N,N'-carbonyldiimidazole, diphenylphosphoryl chloride, diphenylphosphoryl azide, N,N'-disuccinimidyl carbonate, N,N'-disuccinimidyl oxalate, 1-ethyl-3-(3-dimethylaminopropyl)carbodiimide hydrochloride, ethyl chloroformate, isobutyl chloroformate, benzotriazol-1-yl-oxy-tris(dimethylamino)phosphonium hexafluorophosphate, and the like, and among these, thionyl chloride, 1-ethyl-3-(3-dimethylaminopropyl)carbodiimide hydrochloride, N,N-dicyclohexyl carbodiimide, benzotriazol-1-yl-oxy-tris(dimethylamino)phosphonium hexafluorophosphate, and the like are preferable, for example. In the amide-forming reaction, together with the amide-forming reagent, a base and a condensation aid may also be used.

Examples of bases to be used include tertiary aliphatic amines, such as trimethylamine, triethylamine, N,N-diisopropylethylamine, N-methylmorpholine, N-methylpyrrolidine, N-methylpiperidine, N,N-dimethylaniline, 1,8-diazabicyclo[5.4.0]undec-7-ene (DBU), 1,5-azabicyclo[4.3.0]non-5-ene (DBN); aromatic amines and the like, such as pyridine, 4-dimethylaminopyridine, picoline, lutidine, quinoline, isoquinoline, and among these, tertiary aliphatic amines are preferable, and particularly triethylamine, N,N-diisopropylethylamine or the like are preferable.

Examples of condensation aids to be used include N-hydroxybenzotriazole hydrate, N-hydroxysuccinimide, N-hydroxy-5-norbornene-2,3-dicarboxylmide, 3-hydroxy-3,4-dihydro-4-oxo-1,2,3-benzotriazole, or the like, and among these, N-hydroxybenzotriazole and the like are preferable.

A specific example of the compound (2) to be used includes 1-methyl-1H-pyrazol-3-amine, 1-ethyl-1H-pyrazol-3-amine, 1-(1-methylethyl)-1H-pyrazol-3-amine, or the like.

The amount of compound (2) to be used varies depending on the kinds of compound and solvent to be used and other reaction conditions, while the amount is usually 1 to 10 equivalents, and preferably 1 to 3 equivalents, relative to 1 equivalent of the compound (1) or a reactive derivative thereof.

The amount of base to be used varies depending on the kinds of compound and solvent used and other reaction conditions, but the amount is usually 1 to 10 equivalents, and preferably 1 to 5 equivalents.

The reaction solvent to be used in this step is not limited insofar as it does not interfere with the reaction, and may be an inert solvent, for example, and specific examples thereof include methylene chloride, chloroform, 1,2-dichloroethane, dimethylformamide, ethyl acetate ester, methyl acetate ester, acetonitrile, benzene, xylene, toluene, 1,4-dioxane, tetrahydrofuran, dimethoxyethane, and mixed solvents thereof, for ensuring a suitable reaction temperature, methylene chloride, chloroform, 1,2-dichloroethane, acetonitrile, N,N-dimethylformamide, and the like are preferable, for example.

The reaction time is usually 0.5 to 96 hours, and preferably 3 to 24 hours.

The reaction temperature is usually 0°C to the boiling temperature of the solvent, and preferably room temperature to 80°C.

The base, the amide-forming reagent, and the condensation aid used in this step may be one or a combination of two or more kinds.

The thus-obtained compound (3) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 2)

This step is a method of reacting the compound (3) obtained in the above step 1 with a thiol compound (4) in the presence of a base to produce a compound (5).

A specific example of the thiol compound (4) to be used in this reaction is 4-hydroxyphenol, 4-mercaptobenzoic acid, (4-mercaptophenyl)acetic acid, (4-mercaptophenyl)methanol, or the like.

The amount of compound (4) to be used in this step is usually 0.2 to 20 equivalents, and preferably 1 to 10 equivalents, relative to 1 equivalent of the compound (3).

Specific examples of bases to be used in this step include tertiary aliphatic amines such as trimethylamine, triethylamine, N,N-diisopropylethylamine, N-methylmorpholine, N-methylpyrrolidine, N-methylpiperidine, N,N-dimethylaniline, 1,8-diazabicyclo[5.4.0]undec-7-ene (DBU), 1,5-azabicyclo[4.3.0]non-5-ene (DBN); aromatic amines, for example pyridine, 4-dimethylaminopyridine, picoline, lutidine, quinoline, isoquinoline; alkali metals, for example metal potassium, metal sodium, metal lithium; alkali metal hydrides, for example sodium hydride, potassium hydride; alkylated alkali metals, for example butyl lithium; alkali metal alkoxides, for example potassium tert-butoxide, sodium ethoxide, sodium methoxide; alkali metal hydroxides, for example potassium hydroxide, sodium hydroxide; alkali metal carbonates and the like, for example potassium carbonate, sodium carbonate, caesium carbonate, and among these, tertiary aliphatic amines, alkali metal hydrides, alkali metal carbonates, or alkali metal alkoxides are preferable, for example, sodium hydride or potassium carbonate, potassium tert-butoxide, sodium ethoxide or sodium methoxide are particularly preferable.

The amount of base to be used is usually 1 to 10 equivalents, and preferably 1 to 5 equivalents, relative to 1 equivalent of the compound (3).

The reaction solvent to be used in this step is not limited insofar as it does not interfere with the reaction, and is preferably an inert solvent, for example. Specific examples thereof include methylene chloride, chloroform, 1,2-dichloroethane, trichloroethane, dimethylformamide, dimethylacetamide, N-methylpyrrolidone, acetone, tert-butanol, tert-amyl alcohol, ethyl acetate ester, methyl acetate ester, acetonitrile, benzene, xylene, toluene, 1,4-dioxane, tetrahydrofuran, dimethoxyethane, or mixed solvents thereof, and dimethylformamide, dimethylacetamide, N-methylpyrrolidone, acetonitrile, tert-amyl alcohol, and the like are preferable, and N,N-dimethylformamide, dimethylacetamide, N-methylpyrrolidone, acetonitrile, and the like are more preferable.

The reaction time is usually 0.2 to 100 hours, and preferably 1 to 40 hours.

The reaction temperature is usually -20°C to the boiling temperature of the solvent, and preferably 0°C to the boiling temperature of the solvent.

The thus-obtained compound (5) may be isolated and purified by or known isolation/purification means concentration, vacuum concentration, crystallization, solvent extraction, reprecipitation, chromatography, or and then subjected to the next step, or alternatively, may also be subjected to the next step without isolation and purification.

### (Step 3)

This step is a method of reacting the compound (5) obtained in the step 4 with a compound (6) in the presence of a base to produce a compound (I) according to the present invention.

A specific example of the compound (6) to be used in this step is 4-methyl-4H-1,2,4-triazol-3-ylthiol, 4-ethyl-4H-1,2,4-triazol-3-ylthiol, 4-propyl-4H-1,2,4-triazol-3-ylthiol, 4-(1-methylethyl)-4H-1,2,4-triazol-3-ylthiol, or the like.

The amount of compound (6) to be used is usually 0.2 to 20 equivalents, and preferably 1 to 10 equivalents, relative to 1 equivalent of the compound (5).

Examples of bases to be used in this step may be the same as those mentioned in the above step 2, and among these, potassium tert-butoxide or 1,8-diazabicyclo[5.4.0]undec-7-ene (DBU) are preferable.

The amount of base to be used is usually 0.2 to 10 equivalents, and preferably 1 to 5 equivalents, relative to 1 equivalent of the compound (5).

The reaction solvent to be used is not limited insofar as it does not interfere with the reaction, and is preferably an inert organic solvent, for example. Specific examples thereof include methylene chloride, chloroform, 1,2-dichloroethane, trichloroethane, dimethylformamide, dimethylacetamide, N-methylpyrrolidone, acetone, ethanol, isopropanol, tert-butanol, tert-amyl alcohol, ethyl acetate ester, methyl acetate ester, acetonitrile, benzene, xylene, toluene, 1,4-dioxane, tetrahydrofuran, dimethoxyethane, and mixed solvents thereof, and among these, dimethylformamide, N-methylpyrrolidone, or dimethylacetamide are preferable.

The reaction time is usually 0.2 to 100 hours, and preferably 1 to 40 hours. The reaction temperature is usually 0°C to the boiling temperature of the solvent, and preferably room temperature to the boiling temperature of the solvent.

The thus-obtained compound (I) according to the present invention may be isolated and purified by known isolation/purification means concentration, vacuum concentration under, crystallization, solvent extraction, reprecipitation, chromatography.

Further, (1-3) according to the present invention may be produced by the following method, for example.

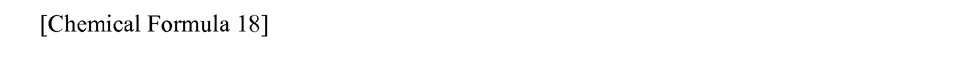

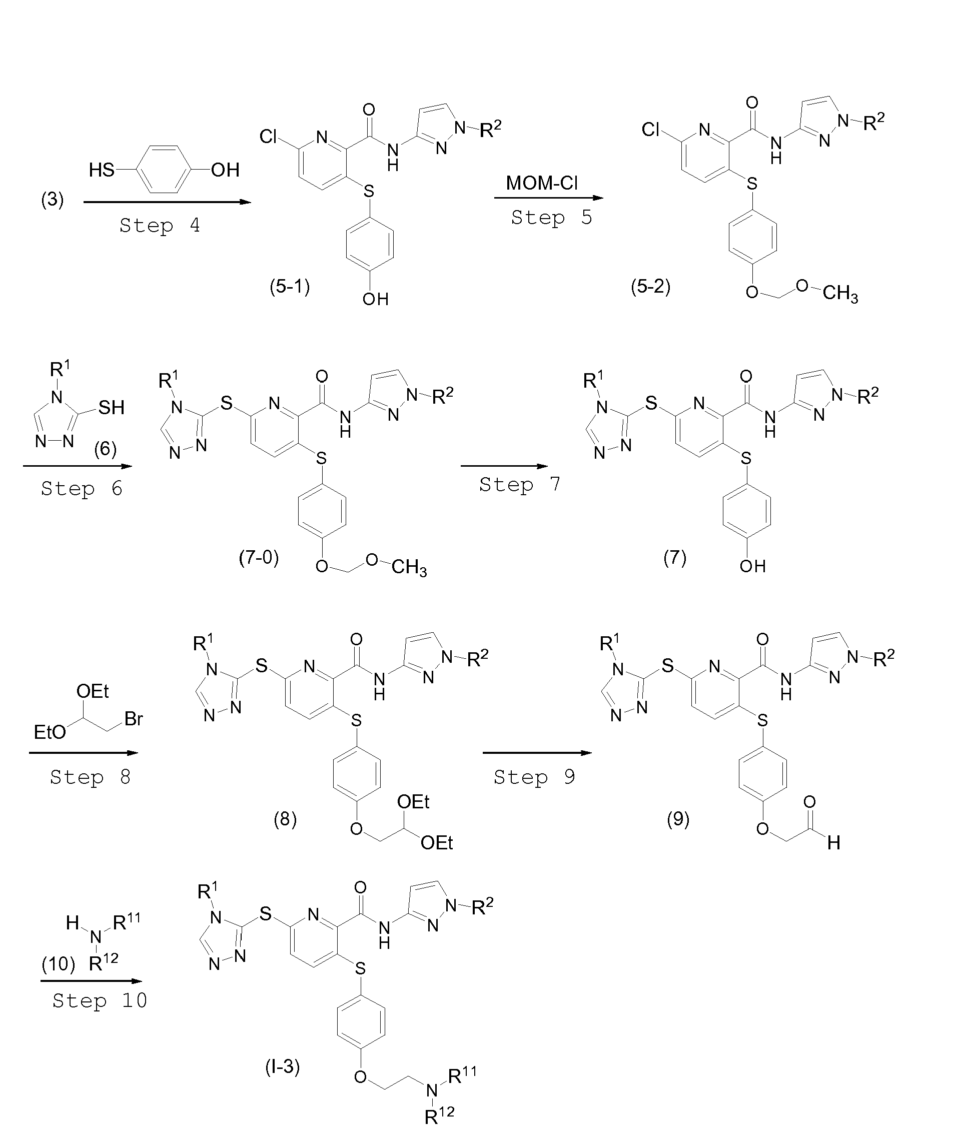

In the formulae, MOM is a methoxymethyl group and the symbols are as defined above.

### (Step 4)

This step is a method of reacting the compound (3) obtained in the above step 1 with 4-hydroxythiophenol in the presence of a base to produce a compound (5-1).

Specific examples of bases to be used in this step include trimethylamine, triethylamine, N,N-diisopropylethylamine, sodium hydride, potassium tert-butoxide, sodium ethoxide or sodium methoxide, potassium hydroxide, sodium hydroxide, potassium carbonate, sodium carbonate, cesium carbonate, and the like, and among these, sodium hydride, potassium tert-butoxide, cesium carbonate, potassium carbonate, and the like are preferable.

The amount of base to be used is usually 0.2 to 10 equivalents, and preferably 1 to 5 equivalents, relative to 1 equivalent of the compound (3).

The amount of 4-hydroxythiophenol to be used is usually 0.2 to 10 equivalents, and preferably 1 to 3 equivalents, relative to 1 equivalent of the compound (3).

The reaction solvent to be used in this step is not limited insofar as it does not interfere with the reaction, examples thereof include methylene chloride, chloroform, 1,2-dichloroethane, trichloroethane, dimethylformamide, dimethylacetamide, N-methylpyrrolidone, acetone, ethanol, isopropanol, tert-butanol, tert-amyl alcohol, ethyl acetate ester, methyl acetate ester, acetonitrile, benzene, xylene, toluene, 1,4-dioxane, tetrahydrofuran, dimethoxyethane, or mixed solvents thereof, and dimethylformamide, dimethylacetamide, N-methylpyrrolidone, acetonitrile, isopropanol, tert-amyl alcohol, and the like are preferable, and N,N-dimethylformamide, dimethylacetamide, N-methylpyrrolidone, acetonitrile, and the like are more preferable.

The reaction time is usually 0.2 to 100 hours, and preferably 1 to 40 hours.

The reaction temperature is usually 0°C to the boiling temperature of the solvent, and preferably room temperature to the boiling temperature of the solvent.

The thus-obtained compound (5-1) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 5)

This step is a method of introducing a methoxymethyl group (also referred to as a MOM group) into the hydroxy group in the compound (5-1) to produce a compound (5-2). The method for introducing a methoxymethyl group may be the above-mentioned method of Protective Groups in Organic Synthesis (T.W Green, 2nd Ed., John Wiley & Sons, 1991, etc.), a method based on the same, or a combination of such methods with an ordinary method, then a methoxymethyl group can thus be introduced. A MOM group can be introduced by reacting a compound (5-1) with MOM-Cl in the presence of diisopropylamine or a like base. The thus-obtained compound (5-2) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 6)

This step is a method of reacting the compound (5-2) obtained in the above step 5 with a compound (6) in the presence of a base to produce a compound (7-0). Specific examples of bases to be used in this step include trimethylamine, triethylamine, N,N-diisopropylethylamine, 1,8-diazabicyclo[5.4.0]undec-7-ene (DBU), 1,5-azabicyclo[4.3.0]non-5-ene (DBN), sodium hydride, potassium tert-butoxide, sodium ethoxide or sodium methoxide, potassium hydroxide, sodium hydroxide, potassium carbonate, sodium carbonate, cesium carbonate, and the like, and among these, 1,8-diazabicyclo[5.4.0]undec-7-ene (DBU) and potassium tert-butoxide are preferable.

The amount of base to be used is usually 0.2 to 10 equivalents, and preferably 1 to 5 equivalents, relative to 1 equivalent of the compound (5-2).

The amount of compound (6) to be used is usually 0.2 to 20 equivalents, and preferably 1 to 10 equivalents, relative to 1 equivalent of the compound (5-2).

The reaction solvent to be used in this step is not limited insofar as it does not interfere with the reaction, examples thereof include methylene chloride, chloroform, 1,2-dichloroethane, trichloroethane, dimethylformamide, dimethylacetamide, N-methylpyrrolidone, acetone, ethanol, isopropanol, tert-butanol, tert-amyl alcohol, ethyl acetate ester, methyl acetate ester, acetonitrile, benzene, xylene, toluene, 1,4-dioxane, tetrahydrofuran, dimethoxyethane, and mixed solvents thereof, and dimethylformamide, dimethylacetamide, N-methylpyrrolidone, acetonitrile, isopropanol, tert-amyl alcohol, and the like are preferable, and dimethylformamide, dimethylacetamide, N-methylpyrrolidone, acetonitrile, and the like are preferable.

The reaction time is usually 0.2 to 100 hours, and preferably 1 to 40 hours.

The reaction temperature is usually -20°C to the boiling temperature of the solvent, and preferably 0°C to the boiling temperature of the solvent.

The thus-obtained compound (7-0) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 7)

This step is a method of removing the MOM group in the compound (7-0) to produce a compound (7). The MOM group may be removed by the above-mentioned method of Protective Groups in Organic Synthesis (T.W Green, 2nd Ed., John Wiley & Sons, 1991, etc.), a method based on the same, or a combination of such methods with an ordinary method. The removal may be performed by, for example, reacting the compound (7-0) with trifluoroacetic acid in chloroform or a like organic solvent.

The thus-obtained compound (7) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 8)

This step is a method of reacting the compound (7) obtained in the above step 7 with bromoacetaldehyde diethyl acetal in the presence of a base to produce a compound (8).

Specific examples of bases to be used in this step include trimethylamine, triethylamine, N,N-diisopropylethylamine, sodium hydride, potassium-tert-butoxide, sodium ethoxide, sodium methoxide, potassium hydroxide, sodium hydroxide, potassium carbonate, sodium carbonate, cesium carbonate, and the like, and among these, sodium hydride, potassium carbonate, cesium carbonate, and the like are preferable.

The amount of base to be used is usually 0.2 to 10 equivalents, and preferably 1 to 5 equivalents, relative to 1 equivalent of the compound (7).

The amount of bromoacetaldehyde diethyl acetal to be used is usually 1 to 10 equivalents, and preferably 1 to 5 equivalents, relative to 1 equivalent of the compound (7).

The reaction time is usually 0.2 to 100 hours, and preferably 1 to 40 hours.

The reaction temperature is usually -20°C to the boiling temperature of the solvent, and preferably 0°C to the boiling temperature of the solvent.

The thus-obtained compound (8) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 9)

This step is a method of hydrolyzing the compound (8) obtained in the above step 8 with acid to produce a compound (9).

Examples of acids to be used include formic acid, hydrochloric acid, acetic acid, trifluoroacetic acid, and the like.

The amount of acid to be used is 1 equivalent to a solvent amount, and preferably 1 to 100 equivalents.

The reaction time is usually 0.2 to 10 hours, and preferably 0.2 to 5 hours.

The reaction temperature is usually 0°C to 60°C, and preferably 0°C to room temperature.

The thus-obtained compound (9) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 10)

This step is a method of reacting the compound (9) obtained in the step 9 with a compound (10) in the presence of a reducing agent to produce a compound (1-3) according to the present invention.

The amount of compound (10) to be used in this step is usually 1 to 10 equivalents, and preferably 1 to 5 equivalents, relative to 1 equivalent of the compound (9).

Examples of reducing agents to be used include sodium triacetoxyborohydride, sodium cyanoborohydride, and the like.

The amount of reducing agent to be used is usually 1 to 10 equivalents, and preferably 1 to 5 equivalents, relative to 1 equivalent of the compound (9).

In addition, zinc chloride, acetic acid, trifluoroacetic acid, magnesium chloride, boron trifluoride, and the like may be added to the reaction system, and the amount thereof is usually 1 to 10 equivalents, and preferably 1 to 3 equivalents, relative to 1 equivalent of the compound (9).

The reaction solvent is not limited insofar as it does not interfere with the reaction, but examples thereof include methanol, ethanol, acetic acid, tetrahydrofuran, chloroform, dichloromethane, and the like. Among these, chloroform, tetrahydrofuran, and the like are preferable.

The reaction time is usually 1 hour to 24 hours, and preferably 1 hour to 8 hours.

The reaction temperature is usually 0°C to 100°C, and preferably 0°C to 40°C.

The thus-obtained compound (1-3) according to the present invention may be isolated and purified by known isolation/purification means concentration, vacuum concentration, crystallization, solvent extraction, reprecipitation, chromatography.

Further, a compound (1-4) according to the present invention represented by:

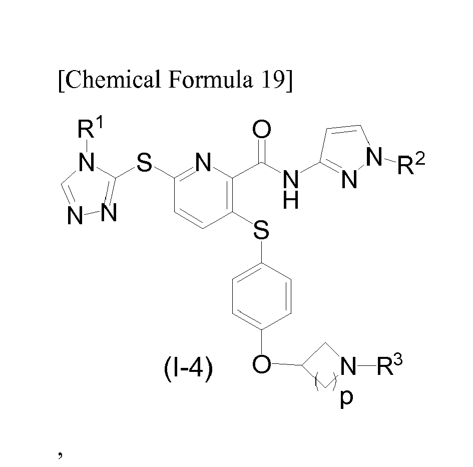

wherein the symbols are as defined above, may also be produced by the following method.

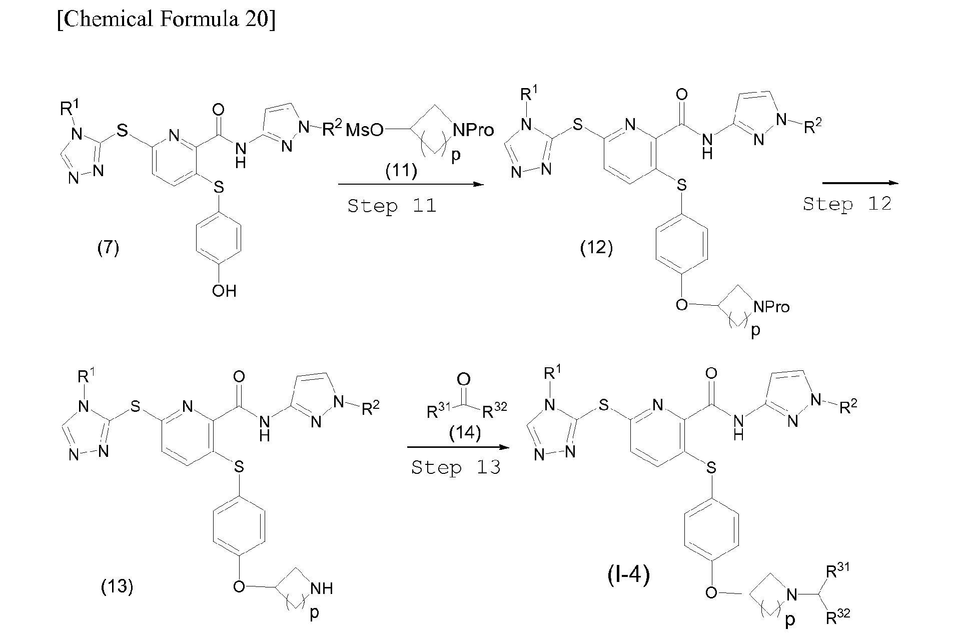

In the formulae, Ms is a methanesulfonyl group, Pro is a protecting group of an amino group, and R³¹ and R³² are each independently a hydrogen atom or a lower alkyl group.

### (Step 11)

This step is a method of reacting the above compound (7) with a compound (11) in the presence of a base to produce a compound (12).

Specific examples of bases to be used in this step include trimethylamine, triethylamine, N,N-diisopropylethylamine, sodium hydride, potassium-tert-butoxide, sodium ethoxide, sodium methoxide, potassium hydroxide, sodium hydroxide, potassium carbonate, sodium carbonate, cesium carbonate, and the like, and among these, sodium hydride, potassium carbonate, cesium carbonate, and the like are preferable.

The amount of base to be used is usually 1 to 10 equivalents, and preferably 1 to 5 equivalents, relative to 1 equivalent of the compound (7).

In place of the methanesulfonyl group in the compound (11), a p-toluenesulfonyl group or a trifluoromethanesulfonyl group may be used.

An example of the compound (11) is 3-[(methylsulfonyl)oxy]-1-azetidinecarboxylic acid 1,1-dimethylethyl ester, 3-[(methylsulfonyl)oxy]-1-pyrrolidinecarboxylic acid 1,1-dimethylethyl ester, or the like.

The amount of compound (11) to be used is usually 1 to 5 equivalents, and preferably 1 to 3 equivalents, relative to 1 equivalent of the compound (7).

The reaction time is usually 10 minutes to 24 hours, and preferably 1 hour to 10 hours.

The reaction temperature is usually 0°C to 150°C, and preferably 0°C to 100°C.

The thus-obtained compound (12) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 12)

This step is a method of removing the amino-protecting group in the compound (12) to produce a compound (13). The reaction in this step is may be performed by the above-mentioned method of Protective Groups in Organic Synthesis (T.W. Green, 2nd Ed., John Wiley & Sons, 1991, etc.), a method based on the same, or a combination of such methods with an ordinary method, and when the amino-protecting group is a Boc group, the protecting group can be removed with hydrochloric acid-dioxane, trifluoroacetic acid, or the like.

The thus-obtained compound (13) may be isolated and purified by known isolation/purification means concentration, vacuum concentration, crystallization, solvent extraction, reprecipitation, chromatography, and then subjected to the next step, or alternatively, may also be subjected to the next step without isolation and purification.

### (Step 13)

This step is a method of reacting the compound (13) with a compound (14) in the presence of a reducing agent to produce a compound (1-4) according to the present invention.

A specific example of the compound (14) is acetone, formaldehyde, acetaldehyde, or the like.

The amount of compound (14) to be used in this step is usually 1 to 10 equivalents, and preferably 1 to 5 equivalents, relative to 1 equivalent of the compound (13).

Examples of reducing agents to be used include sodium triacetoxyborohydride, sodium cyanoborohydride, and the like.

In addition, zinc chloride, acetic acid, trifluoroacetic acid, magnesium chloride, boron trifluoride, and the like may be added to the reaction system, and the amount thereof is usually 1 to 10 equivalents, and preferably 1 to 5 equivalents, relative to 1 equivalent of the compound (13).

The amount of reducing agent to be used is usually 1 to 10 equivalents, and preferably 1 to 5 equivalents, relative to 1 equivalent of the compound (13).

The reaction solvent is not limited insofar as it does not interfere with the reaction, but examples thereof include methanol, ethanol, acetic acid, tetrahydrofuran (THF), chloroform, dichloromethane, a mixed solvent comprising two or more of these, and the like.

The thus-obtained compound (1-4) according to the present invention may be isolated and purified by known isolation/purification means, for example, concentration, vacuum concentration, crystallization, solvent extraction, reprecipitation, chromatography.

In addition, the group represented by the formula:

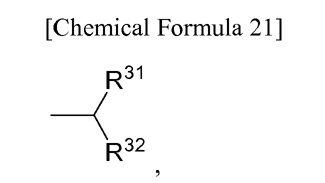

wherein the symbols are as defined above, is as defined with respect to R³ above.

In the reactions mentioned above, when X₁ has a protecting group, then the protective group may be removed according to a method described in references (e.g., Protective Groups in Organic Synthesis, T. W. Green, 2nd Ed., John Wiley & Sons, 1991), a method based on the same, or a combination of such methods with an ordinary method, thereby converting the compound into the compound according to the present invention.

The compound according to the present invention can be produced by the above general production method, a method described in the examples given below, a method based on such methods, or a combination of such methods with an ordinary method.

The 2-pyridinecarboxamide derivative that the present invention provides may be in the form of a pharmaceutically acceptable salt, and the salt may be produced in accordance with an ordinary method using the compound (I) according to the present invention or using a compound expressed by the above formula (I-1), (1-2), (1-3), or (1-4) that is within the scope of the compound (I).

Specifically, when the compound of the Formula (I), (I-1), (1-2), (1-3), or (1-4) has a basic group derived from, for example, an amino group or a pyridyl group in the molecule, then the compound may be processed with acid to convert the same into a corresponding pharmaceutically acceptable salt.

Examples of such acid addition salts include hydrohalides, such as hydrochlorides, hydrofluorides, hydrobromides, hydroiodides; inorganic acid salts, such as nitrates, perchlorates, sulfates, phosphates, carbonates; lower alkylsulfonates, such as methanesulfonates, trifluoromethanesulfonates, ethanesulfonates; arylsulfonates, such as benzenesulfonates, p-toluenesulfonates; organic acid salts, such as fumarates, succinates, citrates, tartrates, oxalates, maleates; and organic acid addition salts with an amino acid, such as glutamates, aspartates. Further, when the compound of the present invention has an acid group in the group, for example, a carboxyl group, then the compound may be processed with a base to convert into a corresponding pharmaceutically acceptable salt. Examples of base addition salts include alkali metal salts, such as sodium salts and potassium salts; alkaline earth metal salts, such as calcium, magnesium; and organic base addition salts, such as ammonium salts, guanidine, triethylamine, dicyclohexylamine, for example. In addition, the compound of the present invention may also be in the form of any hydrate or solvate of a free compound or a salt thereof.

Depending on the aspect of substituent therein, the compound according to the present invention may include a stereoisomer or a tautomer, such as an optical isomer, a diastereoisomer, a geometrical isomer. Needless to say, all such isomers are encompassed by compounds according to the present invention. Further, needless to say, any mixtures of such isomers are also encompassed by compounds according to the present invention.

In the production of medicines for the prevention or treatment of type II diabetes or related diseases or symptoms related thereto, the compound of Formula (I) according to the present invention may be used in combination with a carrier substance.

The dose of the compound of Formula (I) according to the present invention for the prevention or treatment of diseases naturally varies depending on the nature of the symptom to be treated, the specific compound selected, and the administration route.

In addition, the dose also varies depending on the age, body weight, and sensitivity of the patient. In general, the daily dose is, as an amount for single-dose or multiple-dose administration, about 0.001 mg to about 100 mg/kg of body weight, preferably about 0.01 mg to about 50 mg/kg of body weight, and more preferably about 0.1 mg to 10 mg/kg of body weight.
It could be that administration of a dose beyond this range is required.

An example of a suitable dose for oral administration is, for single-dose administration or multiple-dose administration of two to four doses per day, at least about 0.01 mg to at most 2.0 g. Preferably, a daily dose of about 1.0 mg to about 200 mg is administered in one or two doses. More preferably, a daily dose of about 10 mg to 100 mg is administered in a single dose.

For intravenous administration or oral administration, a typical dose is about 0.001 mg to about 100 mg (preferably 0.01 mg to about 10 mg) of the compound of Formula (1)/day/kg of body weight, and more preferably about 0.1 mg to 10 mg of the compound of Formula (1)/day/kg of body weight.

As mentioned above, the pharmaceutical composition comprises a compound of Formula (I) and a pharmaceutically acceptable carrier. The term "composition" includes not only a product obtained by directly or indirectly combining, hybridizing, or aggregating any two or more ingredients, a product obtained as a result of dissociation of one or more ingredients, and a product obtained as a result of reaction or interaction between different types of ingredients, but also an active or inactive ingredient that forms the carrier (pharmaceutically acceptable vehicle).

As combined with a pharmaceutically acceptable carrier, the composition preferably contains a compound of Formula (I) in an amount effective for the treatment or prevention of type II diabetes, or for the delay of its onset.

For administering an effective amount of the compound according to the present invention to mammals, especially to humans, any suitable administration route can be employed. For example, oral administration, rectal administration, local administration, intravenous administration, ophthalmic administration, lung administration, nasal administration, are possible. Examples of dosage forms are tablets, troches, powders, suspensions, solutions, capsules, creams, aerosols. Oral tablets are preferable.

For the preparation of oral compositions, any ordinary pharmaceutical medium is usable, and examples thereof are water, glycol, oil, alcohol, flavoring agents, preservatives, colorants. For the preparation of liquid compositions for oral administration, examples are suspensions, elixirs, and solutions, and as a carrier, for example, starch, sugar, microcrystalline cellulose, a diluent, a granulating agent, a lubricant, a binder, a disintegrant, can be mentioned, and for the preparation of solid compositions for oral administration, examples are powders, capsules, tablets, and above all, such solid compositions for oral administration are preferable.

In view of ease of administration, tablets and capsules are the most advantageous forms for oral administration. If desired, tablets may be coated according to a standard aqueous or non-aqueous coating technique.

In addition to the above-mentioned ordinary dosage forms, the compound according to Formula (I) may also be administered through a release-controlling means and/or delivery system disclosed in U.S. Pat. Nos. 3,845,770, 3,916,899, 3,536,809, 3,598,123, 3,630,200 and 4,008,719, for example.

The pharmaceutical composition according to the present invention suitable for oral administration may be a capsule, a cashew, or a tablet containing a predetermined amount of active ingredient in the form of a powder or granules, or in the form of a water-soluble liquid, a water-insoluble liquid, an oil-in-water emulsion, or a water-in-oil emulsion. Such a composition may be prepared using any pharmaceutical method, but all such methods include a method of combining the active ingredient with a carrier consisting of one or more necessary ingredients.

In general, the active ingredient is uniformly and fully mixed with a liquid carrier, and/or a well-separated solid carrier, or both the two, and then, if desired, the product is shaped into a suitable form, thereby the composition is thus prepared. For example, tablets are prepared through compression and shaping, optionally together with one or more accessory components. Compressed tablets are prepared, using a suitable machine, by mixing an active ingredient optionally with a binder, a lubricant, an inert vehicle, a surfactant, or a dispersant, and then compressing the resulting mixture in any desired manner into a powder or granules.

Shaped tablets are prepared by shaping a mixture of a powdery wet compound and an inert liquid diluent in a suitable machine.

A tablet preferably contains about 1 mg to 1 g of the active ingredient, and a cashew or a capsule contains about 1 mg to 500 mg of the active ingredient.

Examples of the dosage forms of compounds of Formula (I) for pharmaceutical use are as follows:

**TABLE 1**

| Suspension for Injection (I.M.) | |
|---|---|
| | mg/ml |
| Compound of Formula (I) | 10 |
| Methyl cellulose | 5.0 |
| Tween 80 | 0.5 |
| Benzyl alcohol | 9.0 |
| Benzalkonium chloride | 1.0 |
| Injection solvent is added to make 1.0 ml. | |

**TABLE 2**

| Tablet | |
|---|---|
| | mg/tablet |
| Compound of Formula (I) | 25 |
| Methyl cellulose | 415 |
| Tween 80 | 14.0 |
| Benzyl alcohol | 43.5 |
| Magnesium stearate | 2.5 |
| Total | 500mg |

**TABLE 3**

| Capsules | |
|---|---|
| | mg/capsule |
| Compound of Formula (I) | 25 |
| Lactose powder | 573.5 |
| Magnesium stearate | 1.5 |
| Total | 600mg |

**TABLE 4**

| Aerosol | |
|---|---|
| | per can |
| Compound of Formula (I) | 24mg |
| Lecithin, NF Liq. Conc. | 1.2mg |
| Trichlorofluoromethane, NF | 4.025g |
| Dichlorodifluoromethane, NF | 12.15g |

The compound of Formula (I) may be used in combination with other medicines used not only for type II diabetes-related diseases or symptoms but also for treatment/prevention/delay of the onset of type II diabetes. Such other medicines may be administered through an ordinary employed route or at an ordinary dose, simultaneously with or separately from the compound of Formula (I).

In the case where the compound of Formula (I) is used along with one or more other medicines, then a pharmaceutical composition comprising the compound of Formula (I) and such other medicines is preferable. Accordingly, the pharmaceutical composition according to the present invention also comprises, in addition to the compound of Formula (I), one or more other active ingredients. Examples of active ingredients for use in combination with the compound of Formula (I), which may be administered separately or may also be administered as contained in the same pharmaceutical composition, are not limited to those listed in the following (a) to (i):
(a) other glucokinase activators,
(b) biguanides (e.g., buformin, metformin, fenformin),
(c) PPAR agonists (e.g., troglitazone, pioglitazone, nosiglitazone),
(d) insulin,
(e) somatostatin,
(f) α-glucosidase inhibitors (e.g., voglibose, miglitol, acarbose),
(g) insulin secretion promoters (e.g., acetohexamide, carbutamide, chlorpropamide, glibomuride, gliclazide, glymepride, glipizide, glyquidine, glisoxepide, glyburide, glyhexamide, glypinamide, phenbutamide, tolazamide, tolbutamide, tolcyclamide, nateglinide, repaglinide) and
(h) DPP-IV (dipeptidyl peptidase IV inhibitors),
(i) glucose-uptake-promoting agents.

The weight ratio of the compound of Formula (I) to the second active ingredient varies within a broad limit range, and also depends on the effective amount of each active ingredient. Accordingly, for example, when the compound of Formula (I) is used in combination with a PPAR agonist, then the weight ratio of the compound of Formula (I) to the PPAR agonist may be generally about 1000:1 1 to 1:1000, and preferably about 200:1 to 1:200. Combination of the compound of Formula (I) with other active ingredients is made within the above-mentioned range, and in any case, each active ingredient should be used in an effective amount.

The glucokinase-activating effect of the compound according to the present invention and its antihyperglycemic effect based on the same will be demonstrated, for example, by the pharmacological tests given below.

### Pharmacological Experiment 1 (Glucokinase-Activating Effect)

The glucokinase-activating effect of the compound represented by compound (I) according to the present invention and the test method therefor are described below.

The excellent glucokinase-activating effect of the compound represented by the Formula (I) can be determined by a method described in references (e.g., Diabetes, Vol. 45, pp. 1671-1677, 1996, etc.) or by a method based on the same.

The glucokinase activity is determined not by directly measuring glucose-6-phosphate, but by measuring the level of Thio-NADH produced when a reporter enzyme, glucose-6-phosphate dehydrogenase, produces phosphogluconolactone from glucose-6-phosphate.

A recombinant human liver GK used in this assay was expressed in E. coli as a FLAG fusion protein, and then purified by ANTIFLAG M2 AFFIMTY GEL (Sigma).

The assay was carried out using a flat-bottom 96-well plate at 30°C. First, 69 µl of assay buffer (25 mM Hepes Buffer:pH = 7.2, 2 mM MgCl₂, 1 mM ATP, 0.5 mM TNAD, 1 mM dithiothreitol) was dispensed, and 1 µl of DMSO solution of the compound or DMSO as a control was added thereto. Subsequently, 20 µl of enzyme mixture (FLAG-GK, 20 U/ml G6PDH) that had been cooled in ice was dispensed, and 10 µl of 25 mM glucose, a substrate, was added thereto to initiate reaction (final glucose concentration = 2.5 mM).

After the start of the reaction, an increase in the absorbance at 405 nm was measured for 12 minutes at 30 second intervals, and the increment for the first 5 minutes was used for evaluation of the compound. FLAG-GK was added so that the increment of absorbance in 5 minutes in the presence of 1% DMSO was from 0.04 to 0.06.

Taking the OD level of the DMSO control as 100%, the OD level of the test compound at different concentrations was determined. From the OD level at each concentration, Emax (%) and EC50 (µM) were calculated and used as an index of the GK-activating ability of the compound.

The GK-activating ability of the compounds according to the present invention was measured by this method. The results are shown in Table 5 below.

**TABLE 5**

| Compound No. | Emax (%) | EC50 (µM) |
|---|---|---|
| Example 1 | 890 | 0. 12 |
| Example 2 | 1170 | 0. 20 |
| Example 3 | 1060 | 0. 08 |
| Example 4 | 1020 | 0. 07 |
| Example 5 | 1260 | 0. 12 |
| Example 6 | 1020 | 0. 15 |
| Example 7 | 930 | 0. 06 |
| Example 8 | 960 | 0. 08 |
| Example 9 | 880 | 0. 18 |

As shown in the above table, using Emax and EC50 as indicators, the compound according to the present invention has excellent GK-activating ability.

### EXAMPLES

Hereinafter, the present invention will be described in further detail with reference to the Preparation Examples, Examples, and Reference Example; however, the present invention is not limited thereto.

### PREPARATION EXAMPLE 1

Ten parts of the compound of Example 1, 15 parts of heavy magnesium oxide, and 75 parts of lactose are uniformly mixed to give a powdery or granular preparation having a size of 350 µm or less. The preparation is encapsulated to prepare capsules.

### PREPARATION EXAMPLE 2

Forty-five parts of the compound of Example 1, 15 parts of starch, 16 parts of lactose, 21 parts of crystalline cellulose, 3 parts of polyvinyl alcohol, and 30 parts of distilled water are uniformly mixed, ground and granulated, dried, and then sieved to prepare granules having a diameter of 1410 to 177 µm.

### PREPARATION EXAMPLE 3

Granules are prepared in the same manner as in Preparation Example 2, and 3 parts of calcium stearate is added to 96 parts of the granules, and the mixture is shaped under compression to give tablets having a diameter of 10 mm.

### PREPARATION EXAMPLE 4

To 90 parts of granules obtained by the method of Preparation Example 2 are added 10 parts of crystalline cellulose and 3 parts of calcium stearate, and the mixture is shaped under compression to give tablets having a diameter of 8 mm. Subsequently, a mixed suspension of syrup gelatin and precipitated calcium carbonate is applied thereto, thereby preparing sugar-coated tablets.

In the thin-layer chromatography in the Examples, Silicagel 60F₂₄₅ (Merck) was used as the plate, and a UV detector was used for detection. Wakogel™ C-300 (Wako Pure Chemical Industries) was used as the column silica gel, and LC-SORB™ SP-B-ODS (Chemco) or YMC-GEL™ ODS-AQ120-S50 (Yamamura Chemical Laboratories) was used as the reversed-phase column silica gel.

The meanings of the abbreviations in the following examples are as follows.
i-Bu: isobutyl group
n-Bu: n-butyl group
t-Bu: t-butyl group
Me: methyl group
Et: ethyl group
Ph: phenyl group
i-Pr: isopropyl group
n-Pr: n-propyl group
CDCl₃: heavy chloroform group
CD₃OD: heavy methanol group
DMSO-d₆: heavy dimethylsulfoxide group

The meanings of the abbreviations in the nuclear magnetic resonance spectra are as follows.
s: singlet
d: doublet
dd: double doublet
t: triplet
m: multiplet
br:broad
brs: broad singlet
q: quartet
J: coupling constant
Hz: hertz

### REFERENCE EXAMPLE

Synthesis of: 3-[(4-hydroxyphenyl)thio]-N-(1-methyl-1H-pyrazol-3-yl)-6-[(4-methyl-4H-1,2,4-triaz ol-3-yl)thio]pyridine-2-carboxamide

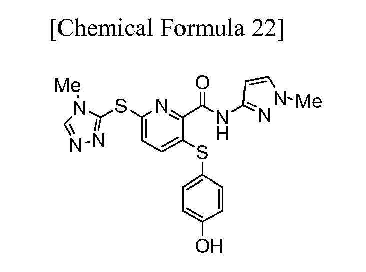

(Step 1) Synthesis of: 3,6-dichloro-N-(1-methyl-1H-pyrazol-3-yl)pyridine-2-carboxamide

To a pyridine (500 ml) solution of 30 g of 3,6-dichloro-2-pyridinecarboxylic acid were successively added 16.7 g of 1-methyl-1H-pyrazol-3-amine and 38.9 g of 1-(3-dimethylaminopropyl)-3-ethylcarbodiimide hydrochloride, and the mixture was stirred at room temperature for 3 hours. Pyridine was distilled off under reduced pressure, and 700 ml of water was added to the obtained residue, and the mixture was stirred for 1 hour to crystallize, thereby giving 36.7 g of the title compound as a pale yellow solid.

(Step 2) Synthesis of: 6-chloro-3-[(4-hydroxyphenyl)thio]-N-(1-methyl-1H-pyrazol-3-yl)pyridine-2-carboxa mide

To a dimethylformamide (600 ml) solution of 33.6 g of 3,6-dichloro-N-(1-methyl-1H-pyrazol-3-yl)pyridine-2-carboxamide were added 20.3 g of 4-hydroxythiophenol and 44.5 g of potassium carbonate under ice-cooling, and the mixture was continuously stirred under ice-cooling for 6 hours, and then stirred at room temperature overnight. Chloroform was added thereto under ice-cooling, and the mixture was washed successively with citric acid solution, water, and saline, and then dried over anhydrous magnesium sulfate. The solvent was distilled off under reduced pressure, and 200 ml of ethyl acetate and 1 L of t-butyl methyl ether were added thereto, and the resulting solid was collected by filtration to give 30.5 g of title compound as a yellow solid.

(Step 3) Synthesis of: 6-chloro-3-[(4-methoxymethoxyphenyl)thio]-N-(1-methyl-1H-pyrazol-3-yl)pyridine-2-carboxamide

To a chloroform (500 ml) solution of 6-chloro-3-[(4-hydroxyphenyl)thio]-N-(1-methyl-1H-pyrazol-3-yl)pyridine-2-carboxa mide were added 7.4 ml of chloromethyl methyl ether and 19.2 ml of diisopropyl ethyl amine under ice-cooling, and the mixture was stirred at room temperature for 6 hours. The reaction solution was washed with aqueous ammonium chloride solution, and dried over anhydrous magnesium sulfate. The solvent was distilled off under reduced pressure, and then the residue was purified by silica gel column chromatography (developing solvent: chloroform) to give 35.1 g of crude purified product of the title compound as a colorless amorphous substance.

(Step 4) Synthesis of: 3-[(4-methoxymethoxyphenyl)thio]-N-(1-methyl-1H-pyrazol-3-yl)-6-[(4-methyl-4H-1, 2,4-triazol-3-yl)thio]pyridine-2-carboxamide

To a dimethylacetamide (170 ml) solution of 17.5 g of 6-chloro-3-[(4-methoxymethoxypheny)thio]-N-(1-methyl-1H-pyrazol-3-yl)pyridine-2-carboxamide were added 24.9 g of 4-methyl-4H-1,2,4-triazole-3-thiol and 32.6 ml of 1,8-diazabicyclo[5.4.0]undec-7-ene, and the mixture was stirred at 120°C for 3 hours. Then, 1 L of chloroform was added thereto at room temperature, washed twice with 500 ml of saturated aqueous ammonium chloride solution, then further washed with water and saturated saline, and dried over anhydrous magnesium sulfate. The solvent was distilled off under reduced pressure to give 20.9 g of the title compound as a brown amorphous substance.

(Step 5) Synthesis of: 3-[(4-hydroxyphenyl)thio]-N-(1-methyl-1H-pyrazol-3-yl)-6-[(4-methyl-4H-1,2,4-triaz ol-3-yl)thio]pyridine-2-carboxamide

To 100 ml of chloroform solution of 20.9 g of 3-[(4-methoxymethoxyphenyl)thio]-N-(1-methyl-1H-pyrazol-3-yl)-6-[(4-methyl-4H-1, 2,4-triazol-3-yl)thio]pyridine-2-carboxamide was added 100 ml of trifluoroacetic acid under ice-cooling, and the mixture was stirred at room temperature overnight. The solvent was distilled off under reduced pressure. The obtained residue was dissolved in 1 L of chloroform and 100 ml of methanol, washed with sodium hydrogen carbonate, and crystallization was performed twice, thereby giving 15.0 g of the title compound as a colorless solid.
¹HNMR(DMSO-d_{b})δ:3.62(3H,s),3.79(3H,s),6.57(1H,d,J=2.1Hz),6.87(2H,d,J=8.8Hz),7 .02(1H,d,J=8.8Hz),7.11(1H,d,J=8.8Hz),7.34(2H,d,J=8.8Hz),7.64(1H,d,J=2.1Hz),8.83( 1H,s),10.03(1H,s),10.07(1H,brs).
ESI-MS(m/e):440[M+H]⁺

### EXAMPLE 1

Synthesis of: 3-({4-[2-(dimethylamino)ethoxy]phenyl}thio)-N-(1-meththy-1H-pyrazol-3-yl)-6-[(4-me thyl-4H-1,2,4-triazol-3-yl)thio]pyridine-2-carboxamide

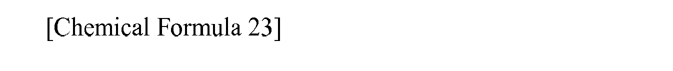

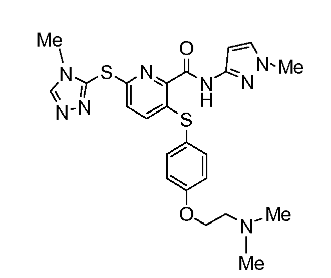

(Step 1) Synthesis of: N-(1-methyl-1H-pyrazol-3-yl)-6-[(4-methyl-4H-1,2,4-triazol-3-yl)thio]-3-{[4-(2-oxoet hoxy)phenyl]thio}pyridine-2-carboxamide

To a dimethylformamide (10 ml) solution of 0.4 g of the 3-[(4-hydroxyphenyl)thio]-N-(1-methyl-1H-pyrazol-3-yl)-6-[(4-methyl-4H-1,2,4-triaz ol-3-yl)thio]pyridine-2-carboxamide obtained in (Step 5) of the Reference Example were added 0.34 ml of bromoacetaldehyde diethyl acetal and 1.33 g of caesium carbonate, and the mixture was stirred at 80°C for 1.5 hours. Saturated aqueous ammonium chloride solution was added thereto at room temperature, followed by extraction with chloroform, and then the organic layer was washed with saturated saline. The washed organic layer was dried over anhydrous magnesium sulfate, and then the solvent was distilled off under reduced pressure. The residue was purified by silica gel column chromatography (developing solvent: chloroform/methanol) to give 0.52 g of yellow solid.

To 0.3 g of the obtained yellow solid were added 0.5 ml of water and 3 ml of trifluoroacetic acid, and the mixture was stirred at room temperature for 30 minutes. The solvent was distilled off under reduced pressure, and then chloroform and saturated saline were added thereto, followed by neutralization with sodium bicarbonate solution. The organic layer was dried over anhydrous magnesium sulfate. The solvent was distilled off under reduced pressure to give 290 mg of the title compound as a yellow solid.

(Step 2) Synthesis of: 3-({4-[2-(dimethylamino)ethoxy]phenyl}thio)-N-(1-methyl-1H-pyrazol-3-yl)-6-[(4-me thyl-4H-1,2,4-triazol-3-yl)thio]pyridine-2-carboxamide

To a tetrahydrofuran solution of 290 mg of the N-(1-methyl-1H-pyrazol-3-yl)-6-[(4-methyl-4H-1,2,4-triazol-3-yl)thio]-3-{[4-(2-oxoet hoxy)phenyl]thio}pyridine-2-carboxamide obtained in Step 1 were added 0.68 ml of dimethyl amine 2M tetrahydrofuran solution and 0.57 mg of sodium triacetoxy borohydride, and the mixture was stirred at room temperature for 30 minutes. Chloroform and saturated saline were added thereto, followed by extraction with chloroform. The organic layer was dried over anhydrous magnesium sulfate, and the solvent was distilled off under reduced pressure, and then the residue was purified by reversed-phase medium-pressure liquid chromatography [ODS-AS-360-CC (manufactured by YMC) mobile phase: water/acetonitrile/0.1% trifluoroacetic acid]. The solvent of the obtained fraction was distilled off under reduced pressure to give the title compound as a trifluoroacetic acid salt. The obtained salt was neutralized, followed by extraction with chloroform, and the organic layer was washed with saturated saline. The washed organic layer was dried over anhydrous magnesium sulfate, and then the solvent was distilled off under reduced pressure, followed by purification by preparative thin-layer chromatography (NH-PLC05 (manufactured by FUJI SILYSIA), chloroform/methanol = 95/5), thereby giving 118 mg of the title compound as a yellow solid.
¹HNMR(CDCl₃)δ:2.45(6H,s),2.76(2H,t,J=5.8Hz),3.73(3H,s),3.86(3H,s),4.10(2H,t,J=5. 8Hz),6.87(1H,d,J=2.0Hz),6.97-7.02(4H,m),7.29(1H,d,J=2.0Hz),7.45(2H,d,J=9.0Hz),8.
42(1H,s),9.87(1H,br)
ESI-MS(m/e):511[M+H]⁺

### EXAMPLE 2

Synthesis of: 3-({4-[2-(diethylamino)ethoxy]phenyl}thio)-N-(1-methyl-1H-pyrazol-3-yl)-6-[(4-meth yl-4H-1,2,4-triazol-3-yl)thio]pyridine-2-carboxamide

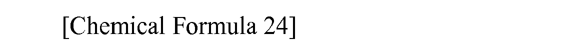

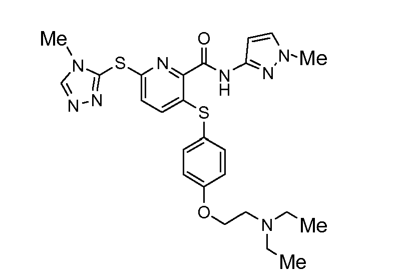

Using diethylamine, the title compound was obtained as a pale yellow solid by the same method as in (Step 2) of Example 1, a method based on the same, or a combination of such methods with an ordinary method.
¹HNMR(CDCl₃)δ:1.08(6H,t,J=7.0Hz),2.65(4H,q,J=7.0Hz),2.90(2H,t,J=6.2Hz),3.73(3 H,s),3.56(3H,s),4.08(2H,t,J=6.2Hz),6.87(1H,d,J=2.0Hz),6.90-7.03(4H,m),7.29(1H,d,J =2.0Hz),7.43(2H,d,J=9.0Hz),8.41(1H,s),9.87(1H,br)
ESI-MS(m/e):539[M+H]⁺

### EXAMPLE 3

Synthesis of: N-(1-methyl-1H-pyrazol-3-yl)-6-[(4-methyl-4H-1,2,4-triazol-3-yl)thio]-3-{[4-(2-pyrrol idin-1-ylethoxy)phenyl]thio}pyridine-2-carboxamide

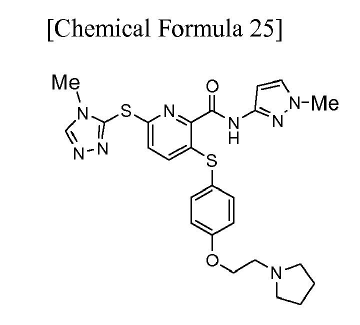

Using pyrrolidine, the title compound was obtained as a colorless solid by the same method as in (Step 2) of Example 1, a method based on the same, or a combination of such methods with an ordinary method.
¹H-NMR(CDCl₃)δ:1.88-1.93(4H,m),2.79-2.86(4H,m),3.07(2H,t,J=5.5Hz),3.73(3H,s),3
.86(3H,s),4.24(2H,t,J=5.5Hz),6.87(1H,d,J=2.3Hz),6.96-7.02(2H,m),6.99(2H,d,J=8.8Hz ),7.30(1H,d,J=2.3Hz),7.45(2H,d,J=8.8Hz),8.42(1H,s),9.88(1H,s) ESI-MS(m/e):537[M+H]⁺

### EXAMPLE 4

Synthesis of: 3-({4-[(1-methylazetidin-3-yl)oxy]phenyl}thio)-N-(1-methyl-1H-pyrazol-3-yl)-6-[(4-methyl-4H-1,2,4-triazol-3-yl)thio]pyridine-2-carboxamide

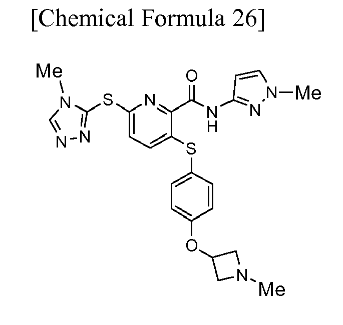

(Step 1) Synthesis of:
t-butyl3-[(methylsulfonyl)oxy]azetidine-1-carboxylate

To a chloroform (25 ml) solution of 4.4 g of t-butyl3-hydroxyazetidine-1-carboxylate were added 3.9 ml o f triethylamine and 2.2 ml of methanesulfonyl chloride under ice-cooling, and the mixture was stirred at room temperature for 40 minutes. Ethyl acetate and saturated aqueous ammonium chloride solution was added thereto at room temperature, followed by extraction with ethyl acetate, and the organic layer was washed with water and saturated saline and dried with anhydrous sodium sulfate. The solvent was distilled off under reduced pressure to give 7.5 g of crude purified product of the title compound as a pale yellow oil.

(Step 2) Synthesis of: 3-{[4-(azetidin-3-yloxy)phenyl]thio}-N-(1-methyl-1H-pyrazol-3-yl)-6-[(4-methyl-4H-1,2,4-triazol-3-yl)thio]pyridine-2-carboxamide

To a dimethylformamide (20 ml) solution of 7.5 g of the t-butyl3-[(methylsulfonyl)oxy]azetidine-1-carboxylate obtained in Step 1 and 8.0 g of the

3-[(4-hydroxyphenyl)thio]-N-(1-methyl-1H-pyrazol-3-yl)-6-[(4-methyl-4H-1,2,4-triaz ol-3-yl)thio]pyridine-2-carboxamide obtained in (Step 5) of the Reference Example was added 17.8 g of caesium carbonate, and the mixture was stirred at 90°C for 4 hours. 1M aqueous citric acid solution was added thereto at room temperature, followed by extraction with chloroform, and then the organic layer was dried over anhydrous sodium sulfate. The solvent was distilled off under reduced pressure, and then the residue was purified twice by silica gel column chromatography (developing solvent: chloroform/methanol) to give 6.3 g of pale orange solid. To the obtained 6.3 g was added 27 ml of 4N hydrogen chloride dioxane solution, and the mixture was stirred at room temperature for 40 minutes. The solvent was distilled off under reduced pressure, then chloroform and aqueous saturated sodium hydrogen carbonate solution were added so that pH = 9, followed by extraction with chloroform, and the organic layer were dried over anhydrous sodium sulfate. The solvent was distilled off under reduced pressure to give 5.3 mg of the title compound as a pale yellow solid.

(Step 3) Synthesis of: 3-({4-[(1-methylazetidin-3-yl)oxy]phenyl}thio)-N-(1-methyl-1H-pyrazol-3-yl)-6-[(4-methyl-4H-1,2,4-triazol-3-yl)thio]pyridine-2-carboxamide

To a 2.0 ml chloroform and 2.0 ml methanol mixed solution of 300 mg of the 3-{[4-(azetidin-3-yloxy)phenyl]thio}-N-(1-methyl-1H-pyrazol-3-yl)-6-[(4-methyl-4H-1,2,4-triazol-3-yl)thio]pyridine-2-carboxamide obtained in Step 2 were added 0.75 ml of 37% aqueous formaldehyde solution and 2.0 ml of 0.3M methanol solution of zinc chloride-sodium cyanotrihydroborate (J. Org. Chem. 1985, 50, 1927-1932), and the mixture was stirred at room temperature for 30 minutes. Aqueous saturated sodium hydrogen carbonate solution and saturated saline were added thereto, followed by extraction with chloroform. The organic layer was dried over anhydrous sodium sulfate, and then the solvent was distilled off under reduced pressure. The residue was purified by reversed-phase medium-pressure liquid chromatography [ODS-AS-360-CC (manufactured by YMC) mobile phase: water/acetonitrile/0.1% trifluoroacetic acid]. The solvent of the obtained fraction was distilled off under reduced pressure, and chloroform was added to the residue, followed by washing with aqueous sodium hydrogen carbonate solution. The organic layer was dried over anhydrous sodium sulfate, then the solvent was distilled off under reduced pressure, and 182 mg of obtained solid was purified by preparative thin-layer chromatography (NH-PLC05 (manufactured by FUJI SILYSIA), chloroform/methanol = 30/1), thereby giving 126 mg of the title compound as a pale yellow solid.
¹H-NMR(CDCl₃)δ:2.43(3H,s),3.13-3.19(2H,m),3.73(3H,s),3.84-3.89(2H,m),3.87(3H,s ),4.75-4.81(1H,m),6.83(2H,d,J=8.6Hz),6.87(1H,d,J=2.3Hz),6.97(1H,d,J=8.6Hz),7.03(1 H,d,J=8.6Hz),7.30(1H,d,J=2.3Hz),7.44(2H,d,J=8.6Hz),8.41(1H,s),9.89(1H,s). ESI-MS(m/e):509[M+H]⁺

### EXAMPLE 5

Synthesis of: 3-({4-[(1-ethylazetidin-3-yl)oxy]phenyl}thio)-N-(1-methyl-1H-pyrazol-3-yl)-6-[(4-met hyl-4H-1,2,4-triazol-3-yl)thio]pyridine-2-carboxamide

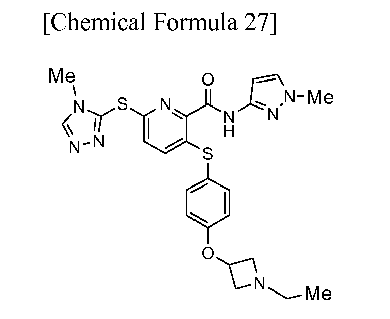

Using the
3-{[4-(azetidin-3-yloxy)phenyl]thio}-N-(1-methyl-1H-pyrazol-3-yl)-6-[(4-methyl-4H-1,2,4-triazol-3-yl)thio]pyridine-2-carboxamide obtained in (Step 2) of Example 4 as a
starting material, and also using acetaldehyde, the title compound was obtained as a colorless solid by the same method as in (Step 3) of Example 4, a method based on the same, or a combination of such methods with an ordinary method.
¹H-NMR(CDCl₃)δ:1.01(3H,t,J=7.2Hz),2.56(2H,q,J=7.2Hz),3.07-3.13(2H,m),3.73(3H,s ),3.81-3.87(2H,m),3.86(3H,s),4.78-4.85(1H,m),6.84(2H,d,J=8.6Hz),6.87(1H,d,J=2.3H z),6.97(1H,d,J=8.6Hz),7.02(1H,d,J=8.6Hz),7.30(1H,d,J=2.3Hz),7.44(2H,d,J=8.6Hz),8. 41(1H,s),9.88(1H,s).
ESI-MS(m/e):523[M+H]

### EXAMPLE 6

Synthesis of: 3-({4-[(1-isopropylazetidin-3-yl)oxy]phenyl}thio)-N-(1-methyl-1H-pyrazol-3-yl)-6-[(4 -methyl-4H-1,2,4-triazol-3-yl)thio]pyridine-2-carboxamide

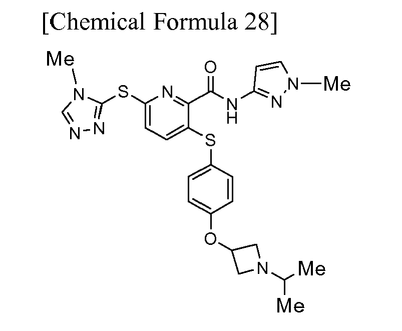

Using the
3-{[4-(azetidin-3-yloxy)phenyl]thio}-N-(1-methyl-1H-pyrazol-3-yl)-6-[(4-methyl-4H-1,2,4-triazol-3-yl)thio]pyridine-2-carboxamide obtained in (Step 2) of Example 4 as a starting material, and also using acetone, the title compound was obtained as a colorless solid by the same method as in (Step 3) of Example 4, a method based on the same, or a combination of such methods with an ordinary method. ¹H-NMR(CDCl₃)δ:0.98(6H,d,J=6.3Hz),2.37-2.44(1H,m),3.09-3.14(2H,m),3.73(3H,s), 3.79-3.87(2H,m),3.86(3H,s),4.75-4.82(1H,m),6.85(2H,d,J=8.6Hz),6.86-6.88(1H,m),6. 97(1H,d,J=8.6Hz),7.03(1H,d,J=8.6Hz),7.26-7.30(1H,m),7.44(2H,d,J=8.6Hz),8.40(1H,s ),9.88(1H,s).
ESI-MS(m/e):537[M+H]⁺

### EXAMPLE 7

Synthesis of: 3-({4-[(1-ethylazetidin-3-yl)methoxy]phenyl}thio)-N-(1-methyl-1H-pyrazol-3-yl)-6-[( 4-methyl-4H-1,2,4-triazol-3-yl)thio]pyridine-2-carboxamide

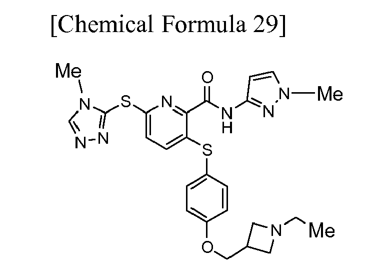

Using the
3-[(4-hydroxyphenyl)thio]-N-(1-methyl-1H-pyrazol-3-yl)-6-[(4-methyl-4H-1,2,4-triaz ol-3-yl)thio]pyridine-2-carboxamide obtained in (Step 5) of the Reference Example and t-butyl3-hydroxymethylazetidine-1-carboxylate, the title compound was obtained as a yellow solid by the same method as in (Step 1-2) of Example 4 and Example 5, a method based on the same, or a combination of such methods with an ordinary method. ¹H-NMR(CDCl₃)δ:0.97(3H,t,J=7.2Hz),2.48(2H,q,J=7.2Hz),2.92(1H,quintet,J=6.8Hz), 3.07(2H,t,J=6.6Hz),3.40(2H,t,J=6.6Hz),3.73(3H,s),3.86(3H,s),4.13(2H,d,J=6.8Hz),6.8 7(1H,d,J=2.3Hz),6.95-7.30(4H,m),7.29(1H,d,J=2.3Hz),7.45(2H,d,J=8.8Hz),8.41(1H,s) ,9.88(1H,br) ESI-MS(m/e):537[M+H]⁺

### EXAMPLE 8

Synthesis of: 3-({4-[(1-isopropylazetidin-3-yl)methoxy]phenyl}thio)-N-(1-methyl-1H-pyrazol-3-yl)-6-[(4-methyl-4H-1,2,4-triazol-3-yl)thio]pyridine-2-carboxamide

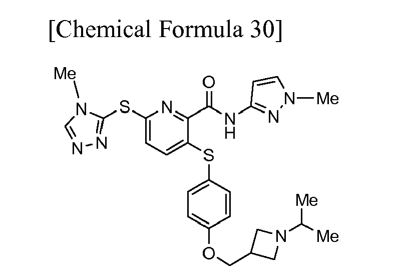

Using acetone, the title compound was obtained as a yellow solid by the same method as in Example 7, a method based on the same, or a combination of such methods with an ordinary method.
¹H-NMR(CDCl₃)δ:0.94(6H,d,J=6.2Hz),2.33(1H,m),2.88(1H,quintet,J=6.6Hz),3.04(2H ,t,J=7.4Hz),3.42(2H,t,J=7.4Hz),3.73(3H,s),3.86(3H,s),4.11(2H,d,J=6.6Hz),6.88(1H,d,J =2.3Hz),6.95-7.03(4H,m),7.30(1H,d,J=2.3Hz),7.45(2H,d,J=8.7Hz),8.41(1H,s),9.88(1H ,br)
ESI-MS(m/e):551[M+H]⁺

### EXAMPLE 9

Synthesis of: 3-({6-[2-(dimethylamino)ethoxy]pyridin-3-yl}thio)-N-(1-methyl-1H-pyrazol-3-yl)-6-[( 4-methyl-4H-1,2,4-triazol-3-yl)thio]pyridine-2-carboxamide

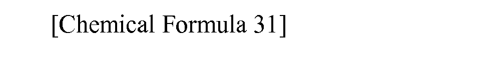

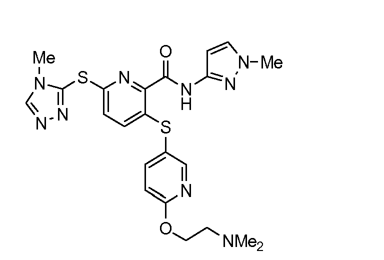

(Step 1) Synthesis of: 6-chloro-3-fluoro-N-(1-methyl-1H-pyrazol-3-yl)pyridine-2-carboxamide

To a pyridine (3 ml) solution of 1.5 g of
6-chloro-3-fluoropyridine-2-carboxylic acid were successively added 1 g of 1-methyl-1H-pyrazol-3-amine and 2.1 g of 1-(3-dimethylaminopropyl)-3-ethylcarbodiimide hydrochloride, and the mixture was stirred at room temperature for 3 hours. Pyridine was distilled off under reduced pressure, water was added to the obtained residue, and stirred for 1 hour, the precipitated solid was collected by filtration, thereby giving 1.6 g of the title compound as a pale yellow solid.

(Step 2) Synthesis of: 6-chloro-3-[(4-methoxybenzyl)thio]-N-(1-methyl-1H-pyrazol-3-yl)pyridine-2-carboxa mide
500 mg of the 6-chloro-3-fluoro-N-(1-methyl-1H-pyrazol-3-yl)pyridine-2-carboxamide obtained in Step 1 was dissolved in 5 ml of dimethylformamide, and 545 mg of (4-methoxypheny)methanethiol and 220 mg of t-butoxy potassium were added thereto,
   and the mixture was stirred at room temperature. Saturated aqueous ammonium chloride solution was added thereto, followed by extraction with chloroform, and then the organic layer was washed with water and saturated saline, and dried with anhydrous sodium sulfate. The solvent was distilled off under reduced pressure, and
   then the residue was dissolved in chloroform, and hexane was added thereto, and the precipitated solid was collected by filtration to give 480 mg of the title compound as a pale yellow solid.

(Step 3) Synthesis of: 2-[(5-iodopyridin-2-yl)oxy]-N,N-dimethylethanamine

To a dimethylformamide (15 ml) solution of 1.5 g of 2-chloro-5-iodopyridine and 0.94 ml of 2-(dimethylamino)ethanol was added 376 mg of 60% sodium hydride under ice-cooling, and the mixture was stirred at room temperature for 30 minutes. Saturated aqueous ammonium chloride solution was added thereto under ice-cooling, followed by extraction with ethyl acetate, and the organic layer was washed with saturated saline. The organic layer was dried over anhydrous sodium sulfate, and then the solvent was distilled off under reduced pressure, and the residue was purified by silica gel column chromatography to give 1.5 g of the title compound was a yellow oil.

(Step 4) Synthesis of: 6-chloro-3-({6-[2-(dimethylamino)ethoxy]pyridin-3-yl}thio)-N-(1-methyl-1H-pyrazol-3-yl)pyridine-2-carboxamide

250 mg of the 6-chloro-3-[(4-methoxybenzyl)thio]-N-(1-methyl-1H-pyrazol-3-yl)pyridine-2-carboxa mide obtained in Step 2 was suspended in 3 ml of trifluoroacetic acid, and 0.067 ml of para-anisole was added thereto, and the mixture was stirred at 60°C for 1.5 hours. The solvent was distilled off under reduced pressure, followed by neutralization with saturated sodium hydrogen carbonate and extraction with chloroform. The organic layer was washed with saturated saline and dried over anhydrous sodium sulfate. The solvent was dried under reduced pressure to give 6-chloro-3-mercapto-N-(1-methyl-1H-pyrazol-3-yl)pyridine-2-carboxamide as a pale brown solid.

A dimethyl sulfoxide (5 ml) suspension of the obtained pale brown solid, 200 mg of 2-[(5-iodopyridin-2-yl)oxy]-N,N-dimethylethanamine, 38 mg of 2-oxocyclohexanecarboxylic acid ethyl ester, 898 mg of caesium carbonate, and 14.8 mg of copper bromide (I) was heated and stirred at 70°C for 3 hours. Saturated ammonium chloride solution was added thereto, followed by extraction with chloroform. The organic layer was washed with saturated saline and dried over anhydrous sodium sulfate, and then the solvent was distilled off under reduced pressure. The residue was purified by silica gel column chromatography to give 240 mg of the title compound as a brown oil.

(Step 5) Synthesis of: 3-({6-[2-(dimethylamino)ethoxy]pyridin-3-yl}thio)-N-(1-methyl-1H-pyrazol-3-yl)-6-[( 4-methyl-4H-1,2,4-triazol-3-yl)thio]pyridine-2-carboxamide

To a dimethylacetamide (1 ml) solution of 80 mg of 6-chloro-3-({6-[2-(dimethylamino)ethoxy]pyridin-3-yl}thio) -N-(1-methyl-1H-pyrazol-3-yl)pyridine-2-carboxamide were added 100 mg of 4-methyl-4H-1,2,4-triazole-3-thiol and 0.14 ml of 1,8-diazabicyclo[5.4.0]undec-7-ene, and the mixture was heated and stirred at 120°C for 3 hours. Chloroform was added thereto, and the mixture was washed with saturated aqueous ammonium chloride solution, water, and saturated saline, and dried over anhydrous sodium sulfate. The solvent was distilled off under reduced pressure, followed by purification by preparative thin-layer chromatography (NH-PLC05 (manufactured by FUJI SILYSIA), chloroform/methanol = 95/5), thereby giving 51 mg of the title compound as a yellow solid.
¹H-NMR(CDCl₃)δ:9.85(1H,s),8.43(1H,s),8.30(1H,d,J=2.3Hz),7.67(1H,dd,J=8.6,2.7H z),7.30(1H,d,J=2.3Hz),7.07(1H,d,J=8.6Hz),7.01(1H,d,J=8.6Hz),6.89(1H,d,J=8.6Hz),6. 86(1H,d,J=2.3Hz),4.46(2H,t,J=5.7Hz),3.86(3H,s),3.74(3H,s),2.74(2H,t,J=5.5Hz),2.34( 6H,s)
ESI-MS(m/e):512[M+H]⁺

### INDUSTRIAL APPLICABILITY

The N-pyrazole-2-pyridinecarboxamide derivative represented by Formula (I) according to the present invention or a pharmaceutically acceptable salt thereof has an excellent glucokinase-activating effect, and thus is, in the medical field, useful in the treatment and/or prevention of diabetes, diabetic complications, or obesity.

## Claims

1. A compound represented by Formula (I) or a pharmaceutically acceptable salt thereof: 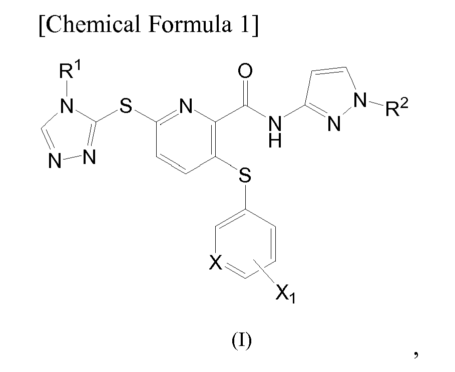 wherein:
R¹ and R² are each independently a lower alkyl group,
X is CH or a nitrogen atom,
X₁ is a group represented by Formula (II-1): 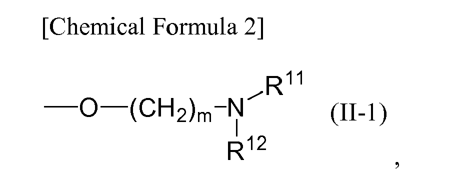
wherein R¹¹ and R¹² are each independently a hydrogen atom or a lower alkyl group; R¹¹, R¹², and the nitrogen atom to which they are bound together form a 4- to 7-membered nitrogen-containing aliphatic ring, wherein one of the carbon atoms that form the 4- to 7-membered nitrogen-containing aliphatic ring may be substituted with an oxygen atom; or alternatively, an arbitrary carbon atom in (CH₂)ₘ and R¹¹ or R¹² together form a 4- to 7-membered nitrogen-containing aliphatic ring, wherein the 4- to 7-membered nitrogen-containing aliphatic ring may be substituted with an oxo group, the nitrogen atom to which R¹¹ and R¹² are bound each other may have an oxygen atom added thereto, an arbitrary carbon atom in (CH₂)ₘ may be substituted with a lower alkyl group, and m is an integer of 1 to 3; or
Formula (II-2): 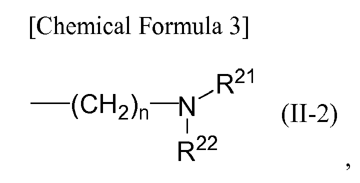 wherein R²¹ and R²² are each independently a hydrogen atom or a lower alkyl group; or alternatively, R²¹, R²², and the nitrogen atom to which they are bound to may together form a 4- to 7-membered nitrogen-containing aliphatic ring, wherein the 4- to 7-membered nitrogen-containing aliphatic ring may be substituted with an oxo group, an arbitrary carbon atom in (CH₂)ₙ may be substituted with a lower alkyl group, and n is an integer of 0 or 1.

2. The compound according to Claim 1 or the pharmaceutically acceptable salt thereof, wherein the compound of Formula (I) is a compound represented by Formula (I-1): 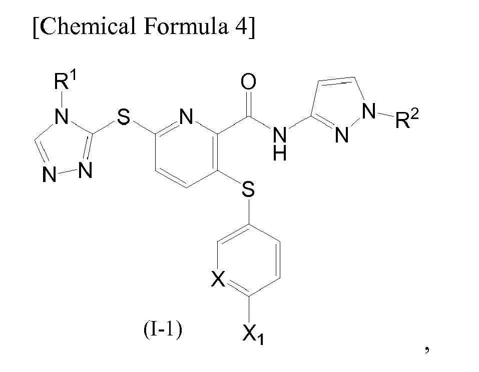 wherein the symbols are as defined above.

3. The compound according to claim 2 or the pharmaceutically acceptable salt thereof, wherein R¹ and R² are both methyl groups.

4. The compound according to claim 3 or the pharmaceutically acceptable salt thereof, wherein X₁ is a group represented by Formula (II-1): 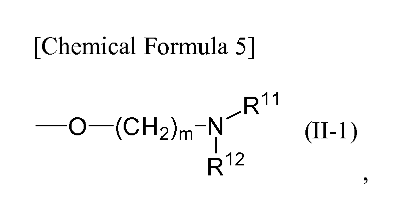 wherein the symbols are as defined above.

5. The compound according to claim 3 or the pharmaceutically acceptable salt thereof, wherein X₁ is a group represented by Formula (II-2): 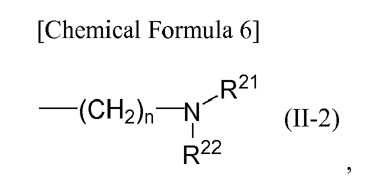 wherein the symbols are as defined above.

6. The compound according to any one of claims 1 to 5 or the pharmaceutically acceptable salt thereof, wherein X is CH.

7. The compound according to any one of claims 1 to 5 or the pharmaceutically acceptable salt thereof, wherein X is a nitrogen atom.

8. The compound according to claim 2 or the pharmaceutically acceptable salt thereof, wherein X is CH and X₁ is represented by Formula (II-1-1): 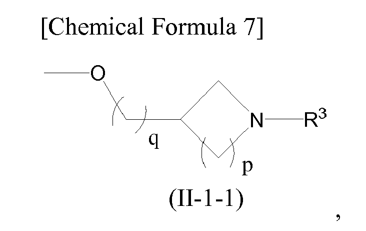 wherein R³ is a lower alkyl group, p is an integer of 1 or 2, and q is an integer of 0 to 2.

9. The compound according to claim 8 or the pharmaceutically acceptable salt thereof, wherein p is 1 and q is 0.

10. The compound according to claim 1 or the pharmaceutically acceptable salt thereof, wherein the compound represented by Formula (I) is:
3-({4-[2-(dimethylamino)ethoxy]phenyl}thio)-N-(1-methyl-1H-pyrazol-3-yl)-6-[(4-methyl-4H-1,2,4-triazol-3-yl)thio]pyridine-2-carboxamide,
3-({4-[2-(diethylamino)ethoxy]phenyl}thio)-N-(1-methyl-1H-pyrazol-3-yl)-6-[(4-methyl-4H-1,2,4-triazol-3-yl)thio]pyridine-2-carboxamide,
N-(1-methyl-1H-pyrazol-3-yl)-6-[(4-methyl-4H-1,2,4-triazol-3-yl)thio]-3-{[4-(2-pyrrolidin-1-ylethoxy)phenyl]thio}pyridine-2-carboxamide,
3-({4-[(1-methylazetidin-3-yl)oxy]phenyl}thio)-N-(1-methyl-1H-pyrazol-3-yl )-6-[(4-methyl-4H-1,2,4-triazol-3-yl)thio]pyridine-2-carboxamide,
3-({4-[(1-ethylazetidin-3-yl)oxy]phenyl}thio)-N-(1-methyl-1H-pyrazol-3-yl)-6-[(4-methyl-4H-1,2,4-triazol-3-yl)thio]pyridine-2-carboxamide,
3-({4-[(1-isopropylazetidin-3-yl)oxy]phenyl}thio)-N-(1-methyl-1H-pyrazol-3 -yl)-6-[(4-methyl-4H-1,2,4-triazol-3-yl)thio]pyridine-2-carboxamide,
3-({4-[(1-ethylazetidin-3-yl)methoxy]phenyl}thio)-N-(1-methyl-1H-pyrazol-3 -yl)-6-[(4-methyl-4H-1,2,4-triazol-3-yl)thio]pyridine-2-carboxamide,
3-({4-[(1-isopropylazetidin-3-yl)methoxy]phenyl}thio)-N-(1-methyl-1H-pyra zol-3-yl)-6-[(4-methyl-4H-1,2,4-triazol-3-yl)thio]pyridine-2-carboxamide,or
3-({6-[2-(dimethylamino)ethoxy]pyridin-3-yl}thio)-N-(1-methyl-1H-pyrazol-3-yl)-6-[(4-methyl-4H-1,2,4-triazol-3-yl)thio]pyridine-2-carboxamide.

11. The compound according to claim 1 or the pharmaceutically acceptable salt thereof, wherein the compound represented by Formula (I) is:
3-({4-[2-(dimethylamino)ethoxy]phenyl}thio)-N-(1-methyl-1H-pyrazol-3-yl)-6-[(4-methyl-4H-1,2,4-triazol-3-yl)thio]pyridine-2-carboxamide.

12. The compound according to claim 1 or the pharmaceutically acceptable salt thereof, wherein the compound represented by Formula (I) is:
3-({4-[2-(diethylamino)ethoxy]phenyl}thio)-N-(1-methyl-1H-pyrazol-3-yl)-6-[(4-methyl-4H-1,2,4-triazol-3-yl)thio]pyridine-2-carboxamide.

13. The compound according to claim 1 or the pharmaceutically acceptable salt thereof, wherein the compound represented by Formula (I) is:
N-(1-methyl-1H-pyrazol-3-yl)-6-[(4-methyl-4H-1,2,4-triazol-3-yl)thio]-3-{[4-(2-pyrrolidin-1-ylethoxy)phenyl]thio}pyridine-2-carboxamide.

14. The compound according to claim 1 or the pharmaceutically acceptable salt thereof, wherein the compound represented by Formula (I) is:
3-({4-[(1-methylazetidin-3-yl)oxy]phenyl}thio)-N-(1-methyl-1H-pyrazol-3-yl )-6-[(4-methyl-4H-1,2,4-triazol-3-yl)thio]pyridine-2-carboxamide.

15. The compound according to claim 1 or the pharmaceutically acceptable salt thereof, wherein the compound represented by Formula (I) is:
3-({4-[(1-ethylazetidin-3-yl)oxy]phenyl}thio)-N-(1-methyl-1H-pyrazol-3-yl)-6-[(4-methyl-4H-1,2,4-triazol-3-yl)thio]pyridine-2-carboxamide.

16. The compound according to claim 1 or the pharmaceutically acceptable salt thereof, wherein the compound represented by Formula (I) is:
3-({4-[(1-isopropylazetidin-3-yl)oxy]phenyl}thio)-N-(1-methyl-1H-pyrazol-3 -yl)-6-[(4-methyl-4H-1,2,4-triazol-3-yl)thio]pyridine-2-carboxamide.

17. The compound according to claim 1 or the pharmaceutically acceptable salt thereof, wherein the compound represented by Formula (I) is:
3-({4-[(1-ethylazetidin-3-yl)methoxy]phenyl}thio)-N-(1-methyl-1H-pyrazol-3 -yl)-6-[(4-methyl-4H-1,2,4-triazol-3-yl)thio]pyridine-2-carboxamide.

18. The compound according to claim 1 or the pharmaceutically acceptable salt thereof, wherein the compound represented by Formula (I) is:
3-({4-[(1-isopropylazetidin-3-yl)methoxy]phenyl}thio)-N-(1-methyl-1H-pyra zol-3-yl)-6-[(4-methyl-4H-1,2,4-triazol-3-yl)thio]pyridine-2-carboxamide.

19. The compound according to claim 1 or the pharmaceutically acceptable salt thereof, wherein the compound represented by Formula (I) is:
3-({6-[2-(dimethylamino)ethoxy]pyridin-3-yl}thio)-N-(1-methyl-1H-pyrazol-3-yl)-6-[(4-methyl-4H-1,2,4-triazol-3-yl)thio]pyridine-2-carboxamide.

20. A pharmaceutical composition for treating, preventing, and/or delaying the onset of type II diabetes, the pharmaceutical composition comprising the following (1) to (3):
(1) the compound according to Claim 1 represented by Formula (I): 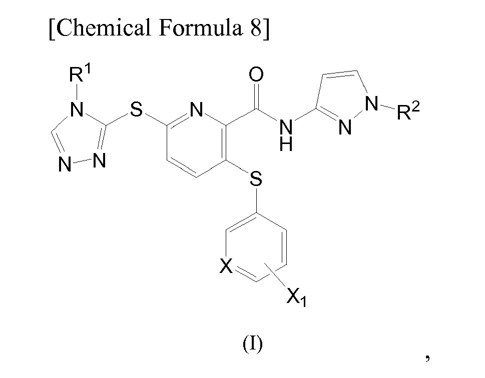 wherein the symbols are as defined above;
(2) one or more compounds selected from the group consisting of the following (a) to (i):
(a) other glucokinase activators,
(b) biguanides,
(c) PPAR agonists,
(d) insulin,
(e) somatostatin,
(f) α-glucosidase inhibitors,
(g) insulin secretion promoters,
(h) DPP-IV inhibitors (dipeptidyl peptidase IV inhibitors), and
(i) glucose-uptake-promoting agents; and
(3) a pharmaceutically acceptable carrier.

21. A glucokinase activator comprising the compound according to any one of claims 1 to 19 or the pharmaceutically acceptable salt thereof, as an active ingredient.

22. A therapeutic agent for diabetes or obesity comprising the compound according to any one of claims 1 to 19 or the pharmaceutically acceptable salt thereof, as an active ingredient.

23. A pharmaceutical composition comprising the compound according to any one of claims 1 to 19 and the pharmaceutically acceptable carrier.
